# EUROPEAN PATENT APPLICATION

(11) **EP 2 305 281 A1**
(43) Date of publication of application: **06.04.2011**
(21) Application number: 09754871.3
(22) Date of filing: 01.06.2009
(51) Int. Cl.: A61K 36/896, A23L 1/30, A61P 3/00, A61P 25/20, A61P 25/24

(54) **COMPOSITION COMPRISING A HOT WATER EXTRACT OF HEMEROCALLIS PLANTS, SAID COMPOSITION BEING EFFECTIVE IN TREATING FATIGUE BY HAVING ANTIDEPRESSANT PROPERTIES OR BY IMPROVING SLEEP**

(30) Priority: 30.05.2008 JP 2008143471; 18.02.2009 JP 2009035746
(71) Applicant: Somnoquest Co., Ltd., Okinawa 901-0152 (JP)
(72) Inventor: YOSHIHARA, Koichi, Nobeoka-shi Miyazaki 882-0865 (JP); EGUCHI, Naomi, Osaka-shi Osaka 532-0025 (JP); DOE, Nobutaka, Akashi-shi Hyogo 673-0028 (JP)
(74) Representative: Denison, Christopher Marcus
(86) International application number: PCT/JP2009/060405
(87) International publication number: WO 2009/145363

(57) **Abstract**

A composition is provided, containing an extract of a plant of the genus *Hemerocallis* as an active ingredient and having antidepressant-like effects or fatigue-relieving effects based on sleep improvement. A composition is also provided, containing a hot-water extract of a plant of the genus *Hemerocallis* as an active ingredient and having antidepressant-like effects or fatigue-relieving effects based on sleep improvement.

## Description

### TECHNICAL FIELD

The present invention relates to a composition containing an extract of a plant of the genus *Hemerocallis* as an active ingredient and having antidepressant-like effects or fatigue-relieving effects based on sleep improvement.

### BACKGROUND ART

*Hemerocallis fulva* var. *sempervirens* is a plant growing wild in subtropical areas and in Okinawa Prefecture and elsewhere in Japan. It belongs to the genus *Hemerocallis* of the family Liliaceae of the order Liliales, and it is also called *Kuwanso* in Okinawa Prefecture. *Hemerocallis fulva* var. *sempervirens* is traditionally said to have effects on insomnia, excitation, irritation, jaundice, mammary tumor, tumor pain, and the like as an agent for blood replenishment or an antasthenic. For example, it has been reported that the product treated by fermentation of the whole plants of *Hemerocallis fulva* var. *sempervirens* has an effect of prolonging the sleep time (see JP Patent Publication (Kokai) No. 2006-62998 A). Also, it has been reported that flowers, white leaf portions (stems), dry powders of roots, or extracts of *Hemerocallis fulva* var. *sempervirens* reduce the number of nighttime awakenings and have an effect against awakening. However, no experimental data have sufficiently demonstrated that the sleep quality is improved by such plant parts or extracts (see JP Patent Publication (Kokai) No. 2007-6804 A).

It has also been reported that an extract of *Hemerocallis fulva* var. *sempervirens* can be used as an agent for suppressing liver damage (see JP Patent Publication (Kokai) No. 2001-10968 A).

### DISCLOSURE OF THE INVENTION

An object of the present invention is to provide a composition containing an extract of a plant of the genus *Hemerocallis* as an active ingredient and having antidepressant-like effects or fatigue-relieving effects resulting from sleep improvement.

It has been traditionally said that *Hemerocallis fulva* var. *sempervirens* has effects on insomnia and the like, but the effects have never been demonstrated. The following is also known concerning *Hemerocallis fulva* var. *sempervirens*: *Hemerocallis fulva* var. *sempervirens* leaves are hard, so that they are not suitable for edible use; the white leaf portions (stems) have an acrid taste; some persons will have loose stools when they eat the white leaf portions (stems); the roots also exert tonic effects in contrast to somnogenic effects; and the active ingredient content in flowers, leaves, white leaf portions (stems), or roots fluctuates depending on seasons, daylight hours, and cultivation environment.

The present inventors have intensively studied the following: the kinds of active ingredients that exist in different parts and the parts of a *Hemerocallis fulva* var. *sempervirens* plant, and the kinds of effects exerted by the active ingredients; and a method for enabling quality control of active ingredients at high predetermined levels regardless of seasonality or the characteristics of the plant-growing area, excluding ingredients or impurities having the aforementioned unfavorable effects of *Hemerocallis fulva* var. *sempervirens,* and formulating active ingredients into easily processable forms as foods or food material.

As a result, the present inventors have discovered that:
ingredients in an extract obtained by hot-water extraction of aboveground parts (mainly leaves and white leaf portions); that is, thermostable and water soluble ingredients that are not denatured by an organic solvent, are active ingredients;
weakly acidic aromatic extracts can be obtained by concentration and extraction, and hot-water extracts mainly obtained from *Hemerocallis fulva* var. *sempervirens* leaves have antidepressant-like effects; and
these active ingredients exert fatigue-relieving effects by improving the sleep quality. Thus, they have completed the present invention.

The present invention is as follows.
[1] A composition having antidepressant-like effects, which contains a hot-water extract of a plant of the genus *Hemerocallis* as an active ingredient.
[2] The composition having antidepressant-like effects according to [1], wherein the plant of the genus *Hemerocallis* is *Hemerocallis fulva* var. *sempervirens.*
[3] The composition having antidepressant-like effects according to [1] or [2], wherein the hot-water extract is a hot-water extract of leaves of a plant of the genus *Hemerocallis.*
[4] The composition having antidepressant-like effects according to any one of [1] to [3], which is a food or a beverage.
[5] The composition having antidepressant-like effects according to [4], which is labeled with its intended use such that it is used for alleviating depressive symptoms.
[6] A composition having fatigue-relieving effects based on sleep improvement, which contains a hot-water extract of a plant of the genus *Hemerocallis* as an active ingredient.
[7] The composition having fatigue-relieving effects based on sleep improvement according to [6], wherein a plant of the genus *Hemerocallis* is *Hemerocallis fulva* var. *sempervirens.*
[8] The composition having fatigue-relieving effects based on sleep improvement according to [6] or [7], wherein the hot-water extract is a hot-water extract of leaves of a plant of the genus *Hemerocallis.*
[9] The composition having fatigue-relieving effects based on sleep improvement according to any one of [6] to [8], which is a food or a beverage.
[10] The composition having fatigue-relieving effects based on sleep improvement according to [9], which is labeled with its intended use such that it is used for relieving fatigue.
[11] A method for producing a composition having antidepressant-like effects and fatigue-relieving effects based on sleep improvement from a plant of the genus *Hemerocallis,* which comprises
   heating a dried plant of the genus *Hemerocallis* with water at 60°C to 100°C for 30 to 90 minutes, filtering the thus obtained extract, and then concentrating the filtrate at 0.5 ml to 2 ml per gram of the dried plant of the genus *Hemerocallis.*
[12] The method according to [11], which further comprises adding 1 to 3 volumes of a water soluble organic solvent to 1 volume of a filtered extract so that the concentration of the organic solvent ranges from 55% to 90%, filtering the resultant, and then concentrating the filtrate to 0.5 ml to 2 ml per gram of the dried plant of the genus *Hemerocallis.*

This description includes part or all of the contents as disclosed in the descriptions and/or drawings of Japanese Patent Application Nos. 2008-143471 and 2009-035746, which are priority documents of the present application.

### BRIEF DESCRIPTION OF THE DRAWINGS

Fig. 1 shows the results of a sensory test for hot-water extracts of *Hemerocallis fulva* var. *sempervirens.*
Fig. 2 shows the sedative effects of a hot-water extract of *Hemerocallis fulva* var. *sempervirens.*
Fig. 3 shows a comparison of the sedative effects of a hot-water extract of *Hemerocallis fulva* var. *sempervirens* with other supplements.
Fig. 4 shows the sedative effects and safety of hot-water extracts of *Hemerocallis fulva* var. *sempervirens.*
Fig. 5 shows the antidepressant-like effects of a hot-water extract of *Hemerocallis fulva* var. *sempervirens.*
Fig. 6 shows the sleep improving effects (regarding amount of sleep) of a hot-water extract of *Hemerocallis fulva* var. *sempervirens.*
Fig. 7 shows the sleep improving effects (regarding frequency of electroencephalogram signals) of a hot-water extract of *Hemerocallis fulva* var. *sempervirens.*
Fig. 8 shows the peripheral effects of a hot-water extract of *Hemerocallis fulva* var. *sempervirens.*
Fig. 9 shows the fatigue-relieving effects of a hot-water extract of *Hemerocallis fulva* var. *sempervirens* based on sleep improvement.
Fig. 10 shows the responses of samplers (who ate jelly containing the hot-water extract of *Hemerocallis fulva* var. *sempervirens*) to the Pittsburgh Sleep Quality Index (questionnaire).
Fig. 11 shows the average sleep time per day over the past month based on responses to the Pittsburgh Sleep Quality Index (questionnaire).
Fig. 12 shows the responses to questions concerning sleepiness.
Fig. 13 shows the effects of a hot-water extract of *Hemerocallis fulva* var. *sempervirens* leaves on an electroencephalogram (subject 1).
Fig. 14 shows the effects of a hot-water extract of *Hemerocallis fulva* var. *sempervirens* leaves on an electroencephalogram (subject 2).
Fig. 15 shows a method for determining antidepressant-like effects.
Fig. 16 shows the antidepressant-like effects of a hot-water extract of *Hemerocallis fulva* var. *sempervirens* leaves and other test substances.
Fig. 17 shows the antidepressant-like effects of a hot-water extract of *Hemerocallis fulva* var. *sempervirens* leaves and other test substances.
Fig. 18 shows the effects of a hot-water extract of *Hemerocallis fulva* var. *sempervirens* leaves and other test substances on deep body temperatures.
Fig. 19 shows the characteristics of a hot-water extract of *Hemerocallis fulva* var. *sempervirens* and other sleep-improving · antianxiety· antidepressive-related substances.

### BEST MODE OF CARRYING OUT THE INVENTION

The present invention is as described more specifically as follows.

Hot-water extracts of plants belonging to the genus *Hemerocallis* of the family Liliaceae of the order Liliales are used. Examples of such plants include *Akinowasuregusa* (*Homerocallis fulva* L. var. *sempervirens* M. Hotta or *Homerocallis sempervirens* Araki), *Honkanzo* (*Hemerocallis fulva* L. var. *fulva*), *Nokanzo* (*Hemerocallis fulva* L. var. *longituba* Maxim or *Hemerocallis longituba* Miq.), and *Yabukanzo* (Hemerocallis fulva L. var. kwanso Regal). Of these examples, *Hemerocallis fulva* var. *sempervirens* (*Kuwanso*) that grows naturally in Okinawa Prefecture, Japan, is preferred. Hot-water extracts may be extracts from whole plants or extracts from plant parts, such as leaves, stems, and roots. In particular, leaves are preferred. Also, leaves include white portions thereof.

The hot-water extracts of the present invention can be obtained by the following method.
(1) Plants are dried. Plants may be dried in the sun or dried using a dryer at 40°C to 120°C.
(2) Extraction is performed from dried plants using host water. Extraction is performed at 60°C to 100°C and the extraction time ranges from 20 to 90 minutes. At this time, water is used accounting for 1% to 5% per weight of a dried plant. As water to be used for extraction, soft water with adjusted minerals, deionized water, or the like is preferably used. A turbid neutral extract displaying dark brownish green color can be obtained by hot-water extraction. Solid matter with a size of 100 meshes or greater or tens of micrometers or greater is removed from the turbid hot-water extract using differences in particle size or gravity. Centrifugation may be performed at a hundred to several thousands × g for several minutes to tens of minutes. The resultant is referred to as a partially purified neutral product obtained by hot-water extraction.
(3) Next, the partially purified neutral product obtained in (2) by hot-water extraction is treated with a water-soluble organic solvent. As a water-soluble organic solvent, ethanol, acetone, methanol, or the like can be used and ethanol is preferably used. The concentration of a water-soluble organic solvent to be used herein ranges from 30% (v/v) to 90% (v/v) with respect to the partially purified neutral product obtained in (2) by hot-water extraction. Upon treatment with a water-soluble organic solvent, the water-soluble organic solvent is preferably added at once to a partially purified neutral product obtained by hot-water extraction, followed by 5 to 60 minutes of stirring. The resultant is left to stand for 6 to 24 hours after stirring at a temperature between a low temperature and room temperature. Treatment with a water-soluble organic solvent and temperature treatment are performed, so that polymers in the partially purified neutral product obtained by hot-water extraction are coagulated and precipitated. Subsequently, the coagulated precipitate in the product treated with the water-soluble organic solvent is removed by filtration or centrifugation. Filtration is performed using cloth, a dedicated cartridge, or the like. Centrifugation may be performed at several hundreds to several tens of thousands × g for several minutes to tens of minutes. A fluid can be obtained by filtration or centrifugation, which has high transparency and is weakly acidic, aromatic, black tea-to-dark orange-based brown colored. This is referred to as a supernatant.
(4) Next, a water soluble organic solvent is removed from the supernatant, from which coagulates have been removed, and then an extract is concentrated. At this time, concentration may be performed using a vacuum evaporator or the like. After concentration, mechanical sterilization is performed using a membrane filter and then finally a weakly acidic hot-water extract can be obtained.

Through hot-water extraction, active ingredients can be extracted, plant compounds with macromolecular structures or plant pigments such as chlorophyll can be removed, and acrid taste or bitterness can be removed from the extract. (5) The partially purified neutral product obtained in (2) by hot-water extraction is subjected to secondary centrifugation and secondary filtration, so that aromatic weakly acidic black tea-to-dark orange-based brown liquid with high transparency can also be obtained. Filtration is performed using dedicated cartridges or dedicated filter paper with a size of several to tens of microns. Centrifugation may be performed at several hundreds to several thousands × g for several minutes to several tens of minutes. Next, the aqueous solution is heated and concentrated at a temperature ranging from 60°C to 90°C for several hours. Through the heating and concentration procedure, finally a weakly acidic hot-water extract is obtained, which is transparent, aromatic, weakly acidic, and orange-based brown-to-dark black tea colored.

Weakly acidic hot-water extracts of the plants of the genus *Hemerocallis* of the present invention, such as hot-water extracts of *Hemerocallis fulva* var. *sempervirens* leaves, white portions of leaf roots, and the like contain a thermostable active ingredient that is not altered by a water soluble organic solvent.

A hot-water extract of a plant of the genus *Hemerocallis* of the present invention has antidepressant-like effects and is useful for prevention or treatment of depression-like symptoms or depressive symptoms. The expression "has (having) antidepressant-like effects" in the present invention refers to alleviation, improvement, or prevention of depression-like symptoms. Whether or not a composition exerts such "antidepressant-like effects" can be determined based on subjective symptoms, diagnosis made by doctors, and the like. Also, it can be confirmed by a water maze test using mice. The extracts of the present invention have antidepressant-like effects and thus can be used as antidepressants as nonprescription drugs, or foods, beverages, or the like having stress-relieving effects or antidepressant-like effects.

Various classifying methods have been proposed for depression. The term "depression" in the present invention refers to, for example: diseases classified as "depression-related disorders" in the "International Classification of Diseases, Injuries and Causes of Death. 10th version (ICD-10)" published by the World Health Organization; or "depression" evaluated by multiaxial diagnosis based on DSM-IV of "Diagnostic Statistic Manual of Mental Disorders (DSM)" according to the American Psychiatric Association (APA).

Also, the hot-water extracts of plants of the genus *Hemerocallis* of the present invention have sleep-improving effects, so that they can be used as agents for sleep improvement, or foods or beverages having sleep-improving effects, for example.

The term "sleep improvement" refers to improvement of sleeplessness or insomnia. Insomnia is a sleep disorder with 4 symptoms: disorder of initiating sleep characterized by having difficulty getting to sleep; awakening characterized by frequent waking up in the middle of the night; early waking up characterized by early waking up followed by sleeplessness; and deep sleep deficiency characterized by being unable to obtain the feeling of deep sleep although sufficient sleep time is ensured. In general, a benzodiazepine hypnotic such as nitrazepam is used for sleep disorders. When a benzodiazepine hypnotic is taken, initiation of sleep is promoted. However, when the frequency of electroencephalogram signals during sleep is examined, characteristic results are obtained, including a decrease in deep non-REM sleep showing a delta band of 0.75 Hz-4 Hz, a decrease in REM sleep showing a theta band of 6 Hz-9 Hz, and the appearance of abnormal fast wave analogous to that in the case of wakefulness state having a peak between 15 Hz and 20 Hz (Michi Sugano, Hirofumi Watanabe, and Kazuko Fuchino, et al., Teikyo Medical Journal, vol. 6, pp311-320, 1983; Hiromi Kawasaki, Koichiro Takahashi: Folia pharmacol. Japon. Vol. 98, pp 259-272, 1991). This means lowered quality of sleep.

Whether or not the weakly acidic hot-water extract of a plant of the genus *Hemerocallis* of the present invention has fatigue-relieving effects for brains can be confirmed by, for example, administering the weakly acidic hot-water extract to an animal and then analyzing bands representing the frequency (appearance) of electroencephalogram signals during sleep and the frequency (appearance (amount)). Deep sleep must be earned for effective fatigue-relieving of cranial nerve.

When the hot-water extract of a plant of the genus *Hemerocallis* of the present invention is ingested before sleep, the deep body temperature is lowered by sedative effects and peripheral vasodilating effects, so as to accelerate the initiation of sleep. Furthermore, in the brain, the hot-water extract increases an electroencephalogram observed in the delta band of non-REM sleep upon the initiation of sleep, decreases light non-REM sleep exhibiting a 11 Hz-14 Hz Sigma band observed in the deep sleep time zone, and has effects of sustaining deep sleep. Also the hot-water extract subsequently increases not only non-REM sleep but also REM sleep, suppressing awakening. Moreover, the hot-water extract prolongs the sleep time. Furthermore, the hot-water extract lowers fatigue upon waking up and alleviates daytime sleepiness after waking up.

The above sleep-related effects of the hot-water extract of a plant of *Hemerocallis fulva* var. *sempervirens* are referred to as sleep-improving effects.

Furthermore, the hot-water extract of a plant of the genus *Hemerocallis* of the present invention has fatigue-relieving effects based on sleep improvement, so that it can be used as a fatigue-relieving agent, a food or a beverage having fatigue-relieving effects, or the like. Here, the term "fatigue" refers to physiological changes induced by extreme exercise or the like and refers specifically to a state in which normal behavior cannot be maintained or a state that is recovered by improvement of nutritional conditions or rest and/or sleep, for example. The hot-water extract of a plant of the genus *Hemerocallis* of the present invention is effective for recovery from these types of fatigue. Also, examples of fatigue include central fatigue and peripheral fatigue. Central fatigue is mental fatigue in the cranial nervous system, which is mainly caused by stresses and the like. On the other hand, peripheral fatigue is caused by muscular movement, which is thought to be mainly caused by energy depletion, accumulation of fatigue substances, and ataxia of internal environment.

The term "fatigue" in the present invention refers to "physiological changes induced by extreme exercise and the like and a state in which normal behavior is not maintained" or "a state that is recovered by improvement of nutritional conditions or rest and/or sleep."

Whether or not the hot-water extract of a plant of the genus *Hemerocallis* of the present invention has fatigue-relieving effects based on sleep improvement can be confirmed by an evaluation method described in Example 4 newly developed by the present inventors.

The term "fatigue-relieving effects (action)" in the present invention refers to effects on physical fatigue induced by forced swimming treatment (Porsolt, R.D., et al, Nature, 266, 730-732, 1977). Forced swimming treatment is recognized as a means for inducing a depressive state. Such depressive state is defined by prolonged duration of immobility response in a forced swimming situation. Antidepressant-like effects (action) are determined based on shortening of the duration of immobility response. However, forced swimming treatment is thought to induce physical fatigue (that is, physiological changes associated with energy consumption due to extreme exercise (swimming)), in addition to depressive response (e.g., response analogous to mental fatigue). Such physical fatigue is expressed as decreased spontaneous activity during a dark period (active phase for mice). Such decreased spontaneous activity is distinguished from depressive response, since it cannot be improved by anti-depressive treatment. Meanwhile, since decreased spontaneous activity is alleviated by sleep-inducing diazepam, but is not affected by wakefulness-inducing caffeine, a decreased level of spontaneous activity is thought to be a state that is improved by sleep but is not affected by wakefulness; that is, a state in which fatigue is induced. The hot-water extract of *Hemerocallis fulva* var. *sempervirens* of the present invention alleviates decreased spontaneous activity induced by forced swimming treatment as in the case of diazepam. However, since diazepam lacks antidepressant-like effects (action), the effects of the two are not the same. The hot-water extract of *Hemerocallis fulva* var. *sempervirens* also alleviates a depressive state induced by forced swimming treatment.

Whether or not the hot-water extract of a plant of the genus *Hemerocallis* of the present invention has fatigue-relieving effects based on sleep improvement can be confirmed by administering the hot-water extract to an animal and then analyzing the electroencephalogram during sleep, for example.

Compositions having antidepressant-like effects, compositions having fatigue-relieving effects based on sleep improvement, or compositions having sleep-improving effects, which comprise the hot-water extract of a plant of the genus *Hemerocallis* of the present invention, are used for mammals including humans. Examples of non-human mammals include, primates such as monkeys, rodents such as rats and mice, sheep, pigs, cattle, cats, and dogs.

Compositions having antidepressant-like effects, compositions having fatigue-relieving effects based on sleep improvement, or compositions having sleep-improving effects, which comprise the hot-water extract of a plant of the genus *Hemerocallis* of the present invention, can be used as antidepressants, fatigue-relieving agents based on sleep improvement, or pharmaceutical compositions that are sleep-improving agents. Such composition may be orally or parenterally administered via intravenous injection, intramuscular injection, subcutaneous administration, rectal administration, transdermal administration, or the like. Such composition having antidepressant-like effects, composition having fatigue-relieving effects based on sleep improvement, or composition having sleep-improving effects may also contain an excipient, a disintegrator, a binder, a lubricant, a colorant, and the like. Examples of an excipient include lactose, dextrose, corn starch, sorbit, and crystalline cellulose. Examples of a disintegrator include starch, sodium alginate, gelatin powder, calcium carbonate, calcium citrate, and dextrin. Examples of a binder include dimethylcellulose, polyvinyl alcohol, polyvinyl ether, methylcellulose, ethyl cellulose, gum Arabic, gelatin, hydroxypropylcellulose, and polyvinylpyrrolidone. Examples of a lubricant include talc, magnesium stearate, polyethylene glycol, and hydrogenated vegetable oil. Also, the above injection preparations can be produced, if necessary, by adding a buffering agent, a pH adjusting agent, a stabilizer, and the like. Examples of an oral preparation include tablets, capsules, powders, granules, and syrups. Examples of a parenteral preparation include an agent for rectal administration, a fat and oil suppository, an aqueous suppository, a transdermal cream pharmaceutical, and transdermal paste. The content of the hot-water extract of a plant of the genus *Hemerocallis* of the present invention in a whole composition differs depending on formulation. In general, the hot-water extract of a plant of the genus *Hemerocallis* accounts for 0.1 % by weight to 50% by weight in the whole composition and preferably an active ingredient in the hot-water extract of a plant of the genus *Hemerocallis* accounts for about 0.1 % by weight to 20% by weight in the whole composition. The dose of the hot-water extract is appropriately determined depending on each case in view of age, body weight and gender of patient, disease, the degree of symptoms, and the like. The hot-water extract may be administered in one to several doses per day.

The hot-water extract of a plant of the genus *Hemerocallis* of the present invention can be used as a food or drink composition by mixing the extract with a food or a beverage. At this time, the hot-water extract may be mixed, so that the content of the hot-water extract of a plant of the genus *Hemerocallis* of the present invention is equivalent to 1 g to 4 g (weight) of dry leaves, in a single dose of food or beverage. The hot-water extract of a plant of the genus *Hemerocallis* of the present invention can also be used as a powdery, granular, liquid, or pasty composition for foods or beverages. Examples of foods and beverages include: beverages such as a soft drink, a lactic acid beverage, a favorite beverage, coffee, green tea, black tea, and oolong tea; confectioneries such as candies, chocolate, biscuits, sweets breads, cakes, sweets made of sticky rice, and rice confectionaries; vegetable and/or fruit processed products, such as a fruit beverage, a vegetable beverage, jams, and pastes; alcohol beverages such as rice wine (sake), distilled spirit, wine, Chinese alcoholic beverage, whiskey, vodka, brandy, gin, rum, alcoholic beverage, beer, cold alcohol drink, fruit wine, and liqueur; dairy products such as yogurt, ice cream, butter, cheese, condensed milk, and milk powder; fat and oil processed products, such as dressing, mayonnaise, tempura oil, and salad oil; seasonings such as soy sauce, sauce, vinegar, sweet cooking rice wine, and dressing-type seasoning; dried foods and beverages such as powder juice, powder soup, instant coffee, instant noodles, instant curry, instant miso soup, a prepared food product, a prepared beverage, and a prepared soup; and grain processed products such as a wheat flour processed food and a starch processed food. Also, the hot-water extract can be used itself as a health food or can be used in the form of tablets or grains prepared by mixing it with other useful ingredients. Moreover, the hot-water extract of a plant of the genus *Hemerocallis* of the present invention can also be used as a raw material for foods or beverages.

Examples of foods or beverages include health beverages, health foods, foods for special health use, beverages for special health use, foods with nutrient functions, beverages with nutrient functions, and health supplements (foods or beverages). Here, the term "foods for specified health use" refers to foods that are ingested for a special health purpose in his or her eating habits and are labeled with its positive effect such that the special health purpose may be achieved by the intake thereof. These foods or beverages may be labeled with their effects of: relieving stresses; relieving frustration; alleviating depressive symptoms; alleviating depression of spirit; relieving fatigue; improving sleep; resolving a lack of sleep; adjusting sleep rhythm; waking up feeling good; prolonging sleep time; lowering feeling of fatigue upon waking up; lessening daytime sleepiness; or adjusting everyday life rhythm, for example.

Furthermore, the present invention encompasses a method for evaluating the fatigue-relieving effects of a test article. Here, fatigue-relieving effects include fatigue-relieving effects based on sleep improvement.

Experimental animals have been conventionally used for evaluating the efficiency of various drugs. For example, a forced swimming test is used for screening for an antidepressant. A method described in Porsolt, R.D., et al, Nature, 266, 730-732, 1977 is known, for example. However, there has been conventionally no known method for evaluating the fatigue-relieving effects of a drug. The present inventors have newly established a method for evaluating the fatigue-relieving effects based on sleep improvement.

Experimental animals to be used for the method are preferably rodents such as mice and rats and particularly mice are preferred. According to the method, first animals are acclimatized under 12-12 hour-cycle light-dark conditions. Next, the animals are forced to swim for 15 minutes or more, preferably 15 minutes to 1 hour, and further preferably 15 minutes to 30 minutes during a light period. At this time, forced swimming is preferably initiated at 6 to 8 hours before the start of a dark period. Subsequently, a test article is administered and then the amount of spontaneous activity during the following dark period is measured.

The water temperature for forced swimming ranges from 20°C to 30°C, preferably ranges from 22°C to 27°C, and is further preferably 24°C±1°C. At this time, a water tank for forced swimming is placed within an experimental chamber where sound insulation and light shielding are possible. The experiment is preferably conducted under light shielding and sound-proof conditions. The term "forced swimming" refers to that animals are forced to swim within a water tank under conditions in which the animals cannot escape. As control animals at this time, animals are left to stand in a water tank containing no water for the time same as that of causing forced swimming.

A test article may be administered after forced swimming for evaluation of fatigue-relieving effects via oral administration or parenteral administration such as intravenous injection, intramuscular injection, intraperitoneal injection, subcutaneous injection, or intradermal injection. The dose may be appropriately determined depending on test article. At this time, water, saline, or the like is administered as a control.

The term "the amount of spontaneous activity" refers to the amount of spontaneous exercise of an animal, including stand up and movement from one location to another, for example. The amount of spontaneous exercise can be found by measuring the number of standing up, the number of movement from one location to another, and the like of an animal. The amount of spontaneous activity can be measured using an automatic measurement system using infrared rays, or the like. An example of such system is Super Mex (Muromachi Kikai Co., Ltd.). The amount of spontaneous activity is measured during a dark period. For example, the amount of spontaneous activity is continuously measured for 12 hours of a dark period.

The average value of the amount of spontaneous activity can be measured every hour, for example, and then analyzed.

When the amount of spontaneous activity is significantly higher than that of a control, it can be determined that the test article has fatigue-relieving effects.

Specifically, the present invention relates to a method for evaluating the fatigue-relieving effects of a test article, comprising forcing animals acclimatized under 12-12-hour-cycle light-dark conditions to swim for 15 minutes or more during a light period, administering a test article, and then measuring the amount of spontaneous activity during the following dark period.

Also, the present invention relates to the above method by which animals are forced to swim at 6 to 8 hours before the start of a dark period.

Also, the present invention relates to the above method wherein the fatigue-relieving effects are fatigue-relieving effects based on sleep improvement.

Also, the present invention relates to the above method, wherein the amount of spontaneous activity is the amount of exercise.

Furthermore, the present invention relates to the above method wherein animals are mice.

The present invention will be described more specifically in reference to the following Examples, but the present invention is not limited to these Examples.

### Example 1 Preparation and evaluation of Hemerocallis fulva var. sempervirens hot-water extracts

### 1. Extraction using an organic solvent

### (1) Hot-water extraction

Dried *Hemerocallis fulva* var. *sempervirens* leaves (120 g) were added to a stainless pot, 1600 ml of ion exchanged water was added, and then the pot was heated at 92°C. The solute percentage at this time (dried leaves/ion reduced water (volume) × 100) was 7.5%. The weight ratio of dried leaves to ion exchanged water was 3 : 40.

When the fluid volume was halved, primary filtration was performed using a 12-mesh stainless sieve and then secondary filtration was performed using a sheet of 50-mesh cotton cloth. The fluid volume after the two instances of filtration was 300 ml and the color of the filtrate was dark brownish green. The filtrate was designated a partially purified aqueous solution.

### (2) Extraction with an organic solvent

60% ethanol (450 ml) was added to 300 ml of the partially purified aqueous solution obtained in (1), followed by 5 minutes of stirring. When a conical tube was used as a vessel, stirring was performed by repeatedly turning the tube upside down. When a beaker was used, the solution was gently stirred using a glass stirrer. After stirring, the resultant was left to stand at room temperature for 6 hours. Subsequently, the solution was filtered using two sheets of 50-mesh cotton cloth, so as to remove coagulates. The filtrate displayed transparent dark orange color.

The filtered supernatant was concentrated using a vacuum evaporator to a volume of 300 ml and then ethanol was removed. Deionized water (30 ml to 100 ml) was added and then concentration using a vacuum evaporator was repeated twice to remove ethanol.

Filtration was performed using filter paper (whatman filter paper (Grade3)). A supernatant was sterilized and filtered using a BTF 0.22-µm filter.

The thus obtained filtrate was designated a *Hemerocallis fulva* var. *sempervirens* hot-water extract.

### 2. Mass extraction without using any organic solvent

Dried *Hemerocallis fulva* var. *sempervirens* leaves (7 kg) were added to a vessel, 250 kg of ion exchanged water was added, and then the vessel was heated at 80°C to 90°C for 60 minutes. The extract was filtered using a stainless filter and then further filtered using 50-mesh cloth. The filtrate was subjected to centrifugation at 7200 rpm for 5 minutes to 20 minutes and then the resultant was filtered using a 10-µm membrane filter. Furthermore, filtration was performed using a 2-µm six-layered filter (Naigai Jyoki Kogyo Co., Ltd.). The resultant was heated and concentrated again, and then 14 kg of a hot-water extract was finally obtained.

### 3. Sensory test

A sensory test was conducted for the thus obtained extracts (a partially purified aqueous solution, a hot-water extract extracted using an organic solvent, and a hot-water extract extracted without using any organic solvent (inorganic solvent)).

Test items were color, odor, transparency, taste, sweetness, umami, sourness, and acrid taste. Also, the intake for obtaining the same effects was calculated and pH was measured.

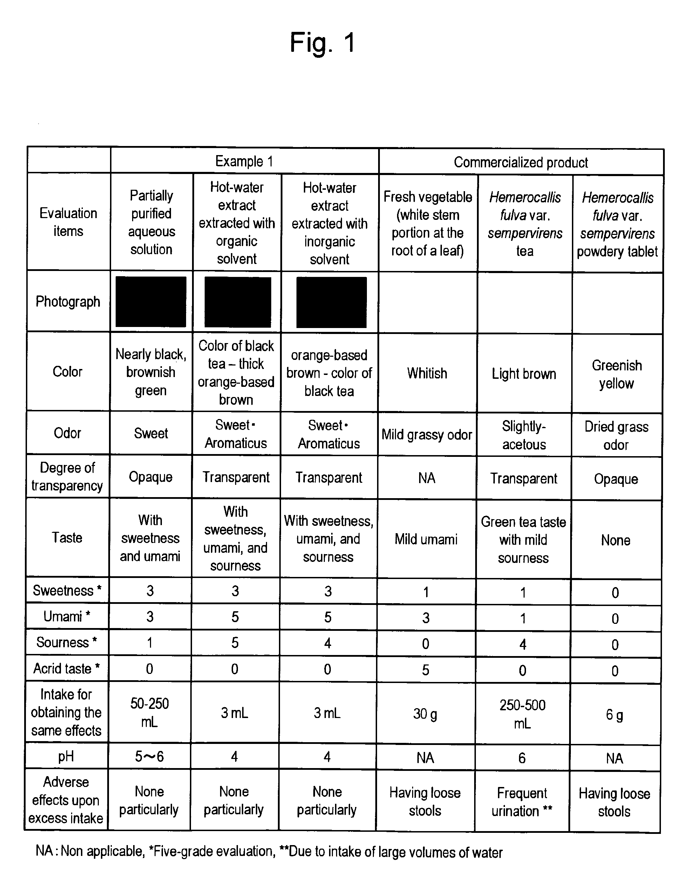

Fig. 1 shows the results.

### Example 2 Sedative effects of Hemerocallis fulva var. sempervirens hot-water extracts

With the use of a system for measuring the amount of spontaneous activity, the effects of Akino (a water soluble ingredient extracted from *Hemerocallis fulva* var. *sempervirens*) on the diurnal variation in the amount of spontaneous activity of mice were examined. Twenty four (24) male ddY mice that had experienced no experiment were bred in an animal room with a constant temperature and humidity for which a 12-12-hour light-dark cycle had been set. Mice were sufficiently acclimatized to breeding environment and then used as subject animals. At the start of the experiment, mice were 10-16-week old and the average body weight was 44.0 g (SD = 3.92). The subject animals were divided into two groups each consisting of 12 mice so that the two groups were almost the same in terms of average body weights and average ages in weeks. One group was designated an Akino group and the other group was designated a control Vehicle group. At 7 hours before the start of a dark period, Akino was orally administered to the Akino group and distilled water was orally administered to the Vehicle group using a stomach tube at 10 ml of a solution per kg mouse body weight. Subsequently, subject mice were put into an acrylic breeding cage (20 cm long × 30 cm wide × 12 cm height) and then left to stand for about 1 hour. At 6 hours before the start of the dark period, the amount of spontaneous activity was continuously measured for 48 hours. The breeding cages used herein were sterilized in advance. All floor mats, feeding stuffs, water supply bottles, and the like used herein were newly prepared. The amount of spontaneous activity was measured using a system for measuring the amount of exercise of experimental animals (Muromachi Kikai Co., Ltd.) using Super Mex Sensor and dedicated software (CompACT AMS ver.3). The interior of the chamber in which breeding cages are placed was set so that the light-dark cycle within the chamber synchronized with the same within the animal room. Breeding cages were installed with water supply tubes. The subject mice of the Akino group could ingest Akino *ad libitum* and the subject mice of the Vehicle group could ingest distilled water *ad libitum.*

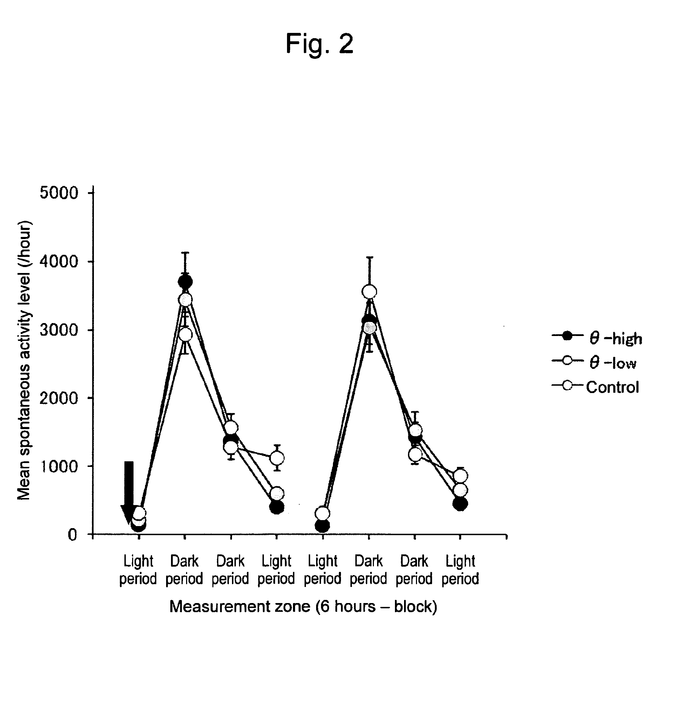

Fig. 2 shows the transition of the amount of spontaneous activity. Fig. 2 shows transition (every 6 hours) of the average amount of spontaneous activity (± standard error) per hour in each group. The amount of spontaneous activity of the Akino group was significantly lower than that of the Vehicle group for 6 hours immediately after transition from the dark period to the light period; that is, in the first half of the dark period. No significant difference was found between the groups in the other measurement zones. Therefore, it was demonstrated that the water soluble ingredient extracted from *Hemerocallis fulva* var. *sempervirens* had effects of decreasing the amount of spontaneous activity; that is, sedative effects only in a light period during which the amount of spontaneous activity of mice was relatively low.

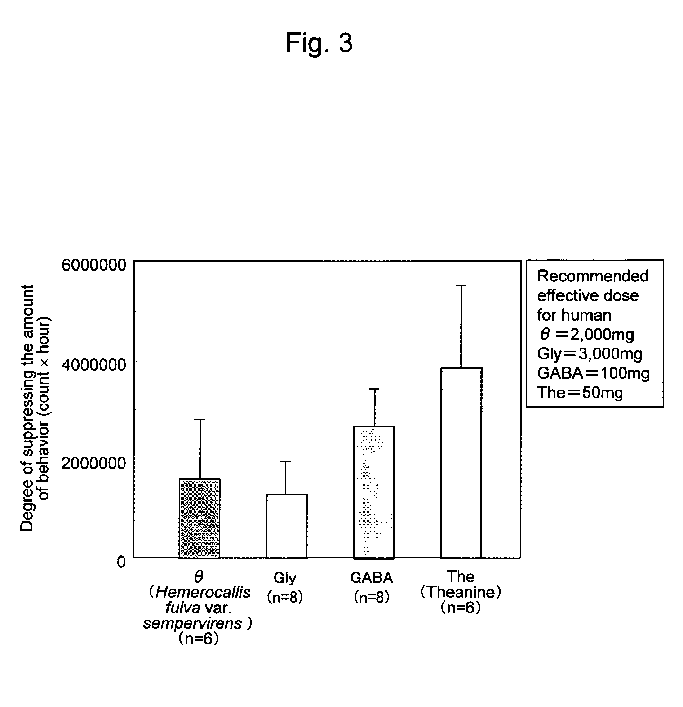

Furthermore, a test of comparing *Hemerocallis fulva* var. *sempervirens* hot-water extract (θ) with other test substances (Gly: glycine, GABA: γ-aminobutyric acid, The: teanin) for sedative effects was conducted. Fig. 3 shows the results. When comparison was made using the same dose (for example, 0.8 g/kg, p.o.), the *Hemerocallis fulva* var. *sempervirens* hot-water extract (θ) exerted sedative effects similar to that exerted by glycine (Gly), GABA (γ-aminobutyric acid), and teanin (The).

### Example 3 Sedative effects and safety of dried Hemerocallis fulva var. sempervirens leaves

Male ddY mice (10-12-week-old) that had experienced no experiment were bred in an animal room with a constant temperature and humidity for which a 12-12-hour light-dark cycle had been set. Mice were sufficiently acclimatized to breeding environment and then used as subject animals. Sterilized acrylic breeding cages (20 cm long × 30 cm wide × 12 cm height) were provided with floor mats, feeding stuffs, and water supply bottles. Regarding feeding stuffs, a feed stuff mixed with *Hemerocallis fulva* var. *sempervirens* dried leaf powder (Akino group) and a non-mixed feed stuff (Cnt group) as a control were prepared one or more times a week and then administered to subject mice. The *Hemerocallis fulva* var. *sempervirens* dried leaf powder-mixed feeding stuff contained an effective concentration of the *Hemerocallis* fulva var. *sempervirens* weakly acidic hot-water extract per 5 g of daily food consumption by mouse.

Measurement of the amount of spontaneous activity was performed for over 6 months using a system for measuring the amount of exercise by an experimental animal using Super Mex Sensor (Muromachi Kikai Co., Ltd.) and dedicated software (CompACT AMS ver.3).

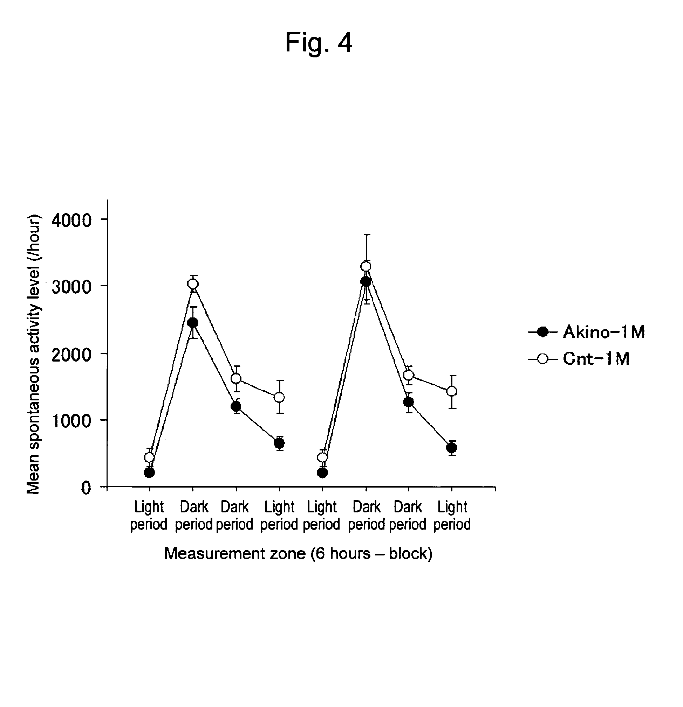

Fig. 4 shows the results of measuring the amount of spontaneous activity after 1 month. Fig. 4 shows the results of 2 days of measurement and specifically shows from the left the average integrated value every 6 hours (±standard error) in the latter half (14-20 o'clock) of a light period, the same in the first half (20-02 o'clock) of a dark period, the same in the latter half (02-08 o'clock), and the same in the first half (08-14 o'clock) of a light period.

The first half of a dark period is the time zone during which mice are the most active. The amounts of spontaneous activity were found to be increased in both the Akino group and the Cnt group. The decreased amount of spontaneous activity of the Akino group was observed in all time zones compared with the Cnt group, but no statistically significant difference was observed in the latter half (14-20 o'clock) of the light period, the first half (20-02 o'clock) of the dark period, and the latter half (02-08 o'clock) of the dark period. Such phenomenon was reproduced during 6 months of administration (the figure is omitted). Therefore, it was considered that dried *Hemerocallis fulva* var. *sempervirens* leaves contained no ingredient composition exerting resistance and accumulation potential.

Meanwhile, the amount of spontaneous activity of the Akino group in the first half (08-14 o'clock) of the light period was statistically significantly decreased compared with that of the Cnt group, demonstrating that mice were sedated. Such a significant decrease was also observed on the next day and was reproduced during 6 months of administration. Therefore, it was considered that mice were sedated before the initiation of the rest time zone (first half of the light period) so that they tended to initiate sleep.

These results revealed that dried *Hemerocallis fulva* var. *sempervirens* leaves had high safety since they exerted no drug-like resistance and accumulation potency even in the case of long-term intake thereof.

### Example 4 Antidepressant-like effects of the Hemerocallis fulva var. sempervirens hot-water extract

An experiment was conducted for determining antidepressant-like effects (action) via application of water-maze learning tasks. Fifty (50) male C57BL/6N mice that had experienced no experiment were bred in an animal room with a constant temperature and humidity for which a 12-12-hour light-dark cycle had been set. Mice were sufficiently acclimatized to breeding environment and then used as subject animals. At the start of the experiment, mice were 10-14-week old and the average body weight was 23.7 g (SD = 3.21). The subject animals were divided into four groups each consisting of 12 or 13 mice so that the groups were almost the same average body weights and average ages in weeks. These groups were designated an OS-Akino group, an OS-SSRI group, an OS-Vehicle group, and a noOS-Vehicle group, respectively. An experimental apparatus was a circular pool coated white having a diameter of 110 cm and a height of 40 cm. The pool was filled with water to the depth of 22 cm. Titanium oxide was added to make water cloudy. The water temperature was maintained at 24°C±1°C.

The experiment was composed of two phases. In phase 1, subject mice of the 3 groups under OS conditions were subjected to open space swimming treatment (OS) for 5 straight days. Specifically, subject mice were released into the pool from a wall once a day and then caused to swim for 5 minutes. In phase 2, a disc-shaped escape platform having a diameter of 11.5 cm was provided within the pool at 5 mm below the water surface, and then training for water maze learning was performed. Specifically, 5 times a day, mice were released into the pool and then the time for the mice to reach the escape platform (escape latency) was measured. When mice did not reach the escape platform within 60 seconds, they were collected and the escape latency was determined to be 60 seconds. Such training for water maze learning was performed for 10 straight days. In phase 2, everyday, at 30 minutes before the start of training for water maze learning, an ingredient (shown in the latter half of the name of each group)-that is, Akino (the water soluble ingredient extracted from *Hemerocallis fulva* var. *sempervirens*), an SSRI (fluvoxamine maleate, 25 mg/kg), or a control vehicle (distilled water)-was administered. Akino and distilled water were orally administered using a stomach tube and an SSRI was intraperitoneally injected. The dose was always 10 ml of the solution per kg mouse body weight.

This example was performed with reference to Sun, M. K., & Alkon, D. L. (2003), Open Space Swimming Test To Index Antidepressant Activity, Journal of Neuroscience Methods, 126, 35-40.

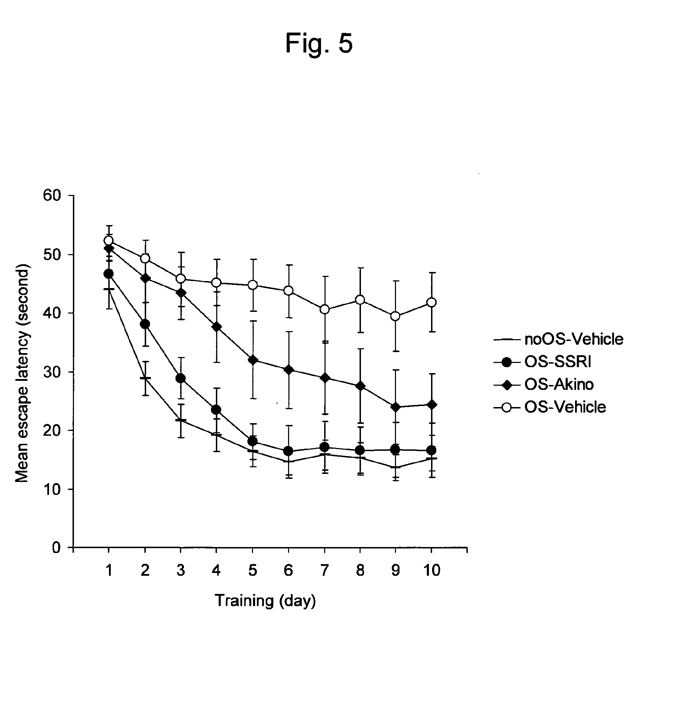

Fig. 5 shows the result of phase 2. Each value in Fig. 5 represents the average escape latency (± standard error) on each training day for each group. In the noOS-Vehicle group, escape latency was rapidly shortened (in terms of a function representing training (day)), indicating that adaptive escape behavior had been acquired. On the other hand, in the OS-Vehicle group, escape latency was almost never shortened and water maze learning disability was clearly observed. This result suggests that a depressive state was induced in subject mice through open space swimming treatment in phase 1, which is interpreted as a state in which motivation was lowered. Such interpretation is also supported by the result of good learning having been achieved by the OS-SSRI group. The escape latency of the OS-Akino group was observed to almost the same degree as with the OS-Vehicle group in the first half of phase 2. However, the escape latency of the OS-Akino group showed a tendency of gradually becoming shorter at the middle of and the latter half of phase 2. On the final day in phase 2, the escape latency of the OS-Akino group reached to a level equivalent to that of the noOS-Vehicle group or the OS-SSRI group. Specifically, it was demonstrated that the water soluble ingredients extracted from *Hemerocallis fulva* var. *sempervirens* have antidepressant-like effects (action) analogous to these of an SSRI.

### Example 5 Sleep-improving effects (action) of the Hemerocallis fulva var. sempervirens hot-water extract

A myoelectric potential electrode was inserted in advance into a neck muscle of a mature male C57BL/6N mouse. An electroencephalogram electrode was attached to the surface of cerebral cortex of each mouse. Mice were placed in a sound proof experimental chamber or box with a constant temperature and humidity (25°C and 55%) for which a 12-12-hour light-dark cycle (lights on at 8:00 and lights off at 20:00) had been set. A wired cable that did not restrict mouse behavior was connected to each of the 2 types of electrode, followed by several days of acclimatization. Electroencephalograms and muscular activity recordings of mice were automatically recorded in real time using dedicated sleep analysis software.

Automatic recording was initiated at 8:00 in the morning (day 1, diurnal variation in basic sleep-wake amount). At 10:00 on the next day, sterile water was orally administered once to unanesthetized mice via a sterilized sonde (day 2, control experiment). The dose was 10 mL per kg of mouse body weight. For example, when the mouse body weight was 30 g, a single dose was 0.3 mL of a solvent. Mice were returned to their original cages after administration, and then action signals of electroencephalogram and myoelectric potential were recorded. At 10:00 on the next day, an *Hemerocallis fulva* var. *sempervirens* weakly acidic hot-water extract (designated with θ in the Figure) was orally administered in the same amount as with the above dose, action signals of electroencephalogram and myoelectric potential were recorded (day 3, sample experiment). Recording was stopped after 48 hours (day 4, recovery experiment).

The thus recorded action potentials of electroencephalogram and myoelectric potential were subjected to digital processing, and then sleep stages of mice were determined at intervals of 4 seconds. Sleep stages were classified into 4 stages: deep non-REM sleep (a 0.75 Hz-4.0 Hz delta band), shallow non-REM sleep (an 11 Hz-14 Hz Sigma band), REM sleep (a 6 Hz-9 Hz theta band), and wakefulness (a 15 Hz-25 Hz) based on a combination of electroencephalogram frequency (Hz), amplitude, and the degree of muscle tone of myoelectric potential. An integrated value was calculated for each stage, corresponding to frequencies (number of times observed during 2 hours) of electroencephalogram signals and then the values were plotted to draw a line graph.

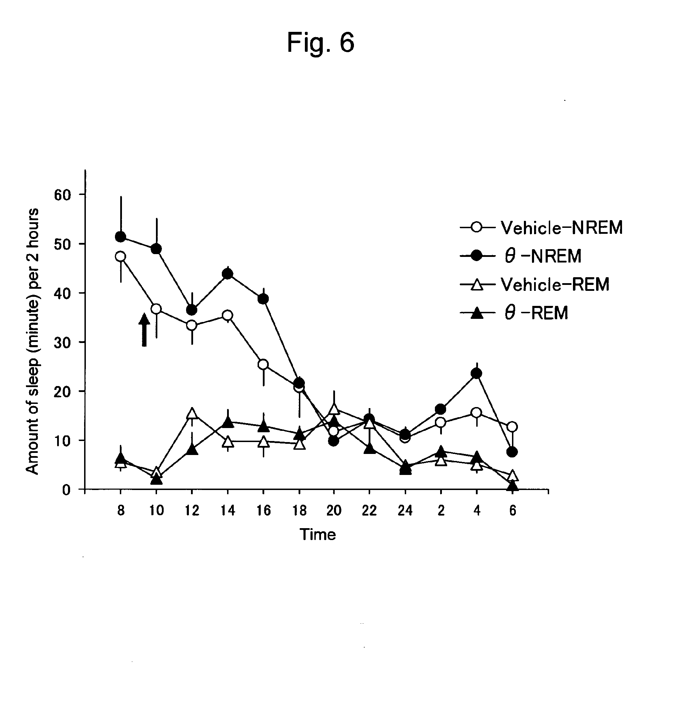

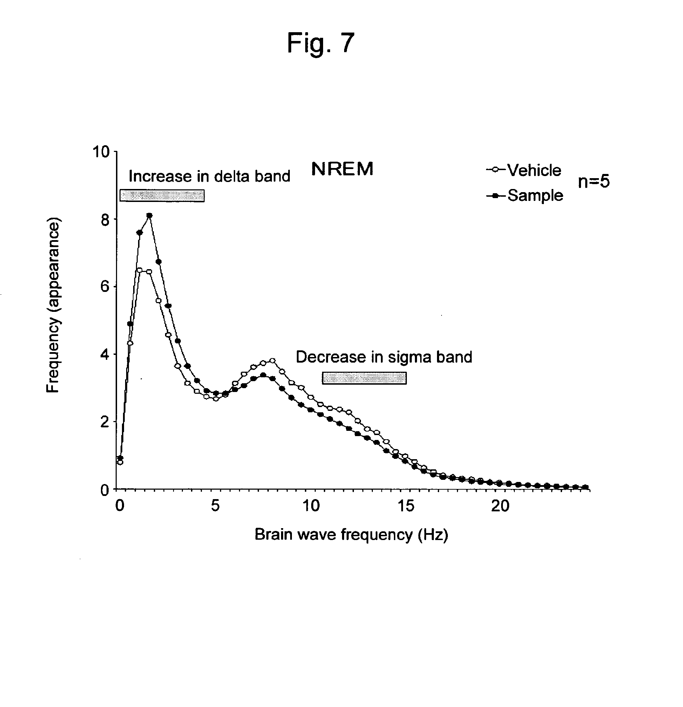

The *Hemerocallis fulva* var. *sempervirens* weakly acidic hot-water extract (n = 5) administered to mice during the rest time zone (8:00-20:00) increased the amount of non-REM sleep to a level higher than that of the mouse group to which the solvent had been administered (n = 5) and allowed such level to be maintained for about 3 hours. Subsequently, the *Hemerocallis fulva* var. *sempervirens* weakly acidic hot-water extract increased both the amount of non-REM sleep and the amount of REM sleep by about 5 hours (Fig. 6). Moreover, the *Hemerocallis fulva* var. *sempervirens* weakly acidic hot-water extract maintained the depth of non-REM sleep for 6 to 9 hours after administration (Fig. 7).

Such increasing-phenomena were not observed even when the extract was administered to mice during the active time zone (20:00-8:00).

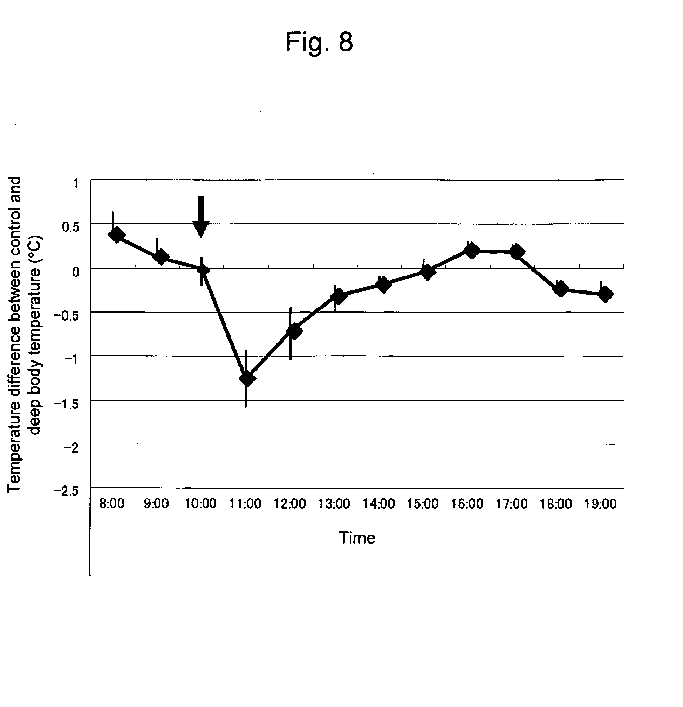

Next, to examine the peripheral effects of the *Hemerocallis fulva* var. *sempervirens* weakly acidic hot-water extract, the *Hemerocallis fulva* var. *sempervirens* weakly acidic hot-water extract was orally administered (arrow) at 10:00 in the morning, 2 hours after the start of the rest time zone. Mouse rectal temperature was measured every hour using a dedicated sensor. The difference between the mouse deep body temperature and the rectal temperature represents a temperature difference compared with that of a control (X ± SE, n = 13; Fig. 8). As a result, when the *Hemerocallis fulva* var. *sempervirens* weakly acidic hot-water extract was administered, peripheral blood vessels of extremities were dilated and became reddish at 30 minutes after administration, and decreased deep body temperatures were also observed. At 1 hour after administration, the temperature reached the lowest temperature peak and then gradually returned to the original base temperature.

Based on the above results, it was considered that the *Hemerocallis fulva* var. *sempervirens* weakly acidic hot-water extract has effects of accelerating the initiation of sleep via body temperature decrease associated with peripheral vasodilating effects after administration, following which increases in the amount of non-REM sleep or REM sleep in the center are maintained.

### Example 6 Fatigue-relieving effects of the Hemerocallis fulva var. sempervirens hot-water extract

An experiment for determining the fatigue-improving effects (action) was conducted using a forced swimming test for mice and a system for measuring the amount of spontaneous activity. Sixty eight (68) male C57BL/6N mice that had experienced no experiment were bred in an animal room with a constant temperature and humidity for which a 12-12-hour light-dark cycle had been set. Mice were sufficiently acclimatized to breeding environment and then used as subject animals. At the start of the experiment, mice were 10-12-week old and the average body weight was 22.8 g (SD = 1.28). The subject animals were divided into seven groups so that the groups were almost the same in terms of average body weights and average ages in weeks. These groups were designated an FS-Akino group (n = 11), an FS-Vehicle group (n = 10), a noFS-Vehicle group (n = 9), an FS-Dia group (n = 9), an FS-Caf group (n = 9), an FS-Imi group (n = 11), and an FS-SSRI group (n = 9).

A cylindrical water tank (with the bottom surface having a diameter of 18 cm and a height of 29 cm) placed in an experimental chamber capable of achieving sound proof and light shielding was filled with water at 24±1°C to a depth of 15 cm. At 7 hours before the start of a dark period, subject mice under FS conditions were subjected to 30 minutes of forced swimming treatment in the tank. Specifically, subject mice were forced to swim under conditions in which they could not escape. Subject mice of the control noFS-Cnt group were left to stand within the same type of empty water tank for 30 minutes.

After 30 minutes of forced swimming treatment, Akino (the water soluble ingredient extracted from *Hemerocallis fulva* var. *sempervirens*), diazepam (Dia, 2.5 mg/kg), caffeine (Caf, 15mg/kg), imipramine (Imi, 30 mg/kg), SSRI (fluvoxamine maleate, 25 mg/kg), or Vehicle (distilled water), which is an ingredient, corresponding to the latter half of each group name, were specifically administered to each subject mouse. Akino and distilled water were orally administered using a stomach tube, and diazepam, caffeine, imipramine, and SSRI were injected intraperitoneally. The dose was always 10 ml of solution per kg mouse body weight.

After administration of these drugs, subject mice were placed in acrylic cages (20 cm long × 30 cm wide × 12 cm high). The amount of spontaneous activity (in the dark period that began at 6 hours after administration) was continuously measured for 12 hours. The breeding cages used herein were sterilized in advance. All floor mats, feeding stuffs, water supply bottles, and the like used herein were newly prepared. The amount of spontaneous activity was measured using a system for measuring the amount of exercise of experimental animals (Muromachi Kikai Co., Ltd.) using Super Mex Sensor and dedicated software (CompACT AMS ver.3).

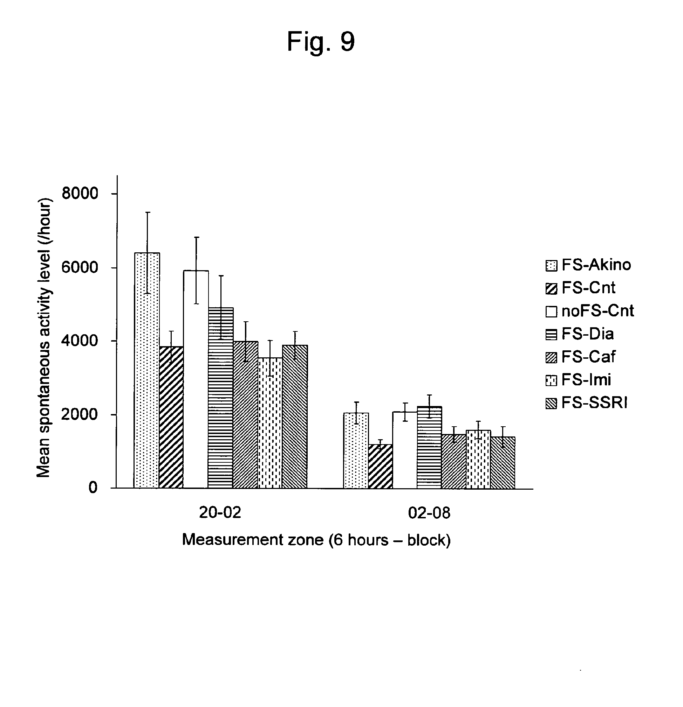

Fig. 9 shows the results of measuring the amounts of spontaneous activity. The left half of Fig. 9 shows the average amount of spontaneous activity (± standard error) per hour of each group in the first half (20-02 o'clock) of the dark period. The right half of Fig. 9 shows the same in the latter half (02-08 o'clock) of the dark period. A dark period is a time zone during which mice are active. It is known that the amount of spontaneous activity is particularly high in the first half of a dark period. As is clear from comparison between the FS-Vehicle group and the noFS-Vehicle, spontaneous activity was significantly suppressed by forced swimming treatment. Also in the FS-Caf group, the FS-Imi group, and the FS-SSRI group, the amount of spontaneous activity was equivalent to that of the FS-Vehicle group and was significantly lower than that of the no FS-Vehicle group. On the other hand, the amount of spontaneous activity of the FS-Dia group was higher than that of the FS-Vehicle group in both the first half and the latter half time zones. The amount of spontaneous activity of the FS-Dia group was at a level near the level of the noFS-Vehicle group. Diazepam is used as a sleeping pill and is understood that it induces sleep after forced swimming treatment, so that spontaneous activity at a decreased level was recovered. A decrease in spontaneous activity, which had been induced by forced swimming treatment, could be improved by sleep. Thus, this is thought to be an index for fatigue.

The amount of spontaneous activity of the FS-Akino group was higher than that of the FS-Vehicle group, as in the case of the FS-Dia group, and was observed at the same level as that of the noFS-Vehicle group. Therefore, it was concluded that the water soluble ingredient extracted from *Hemerocallis fulva* var. *sempervirens* mediates the sleep-improving effects and having effects of alleviating fatigue.

### Example 7 Sleep-improving effects (action) of an Hemerocallis fulva var. sempervirens hot-water extract on humans

The effects of an extract obtained from dried *Hemerocallis fulva* var. *sempervirens* leaves (*Hemerocallis fulva* L. var. *sempervirens M Hotta*) on night-time sleep, next-day physical conditions, and daytime sleepiness of healthy adults were examined by double-blind comparative study (double blind crossover test).

### Materials and methods

Subjects were selected from among 35 applicants who had applied for a "Monitoring Test for Sleep-Improving Supplements" planned by Somnoquest Co., Ltd., excluding applicants who were getting outpatient treatment because of some diseases such as sleep disorder and applicants who took some drugs or supplements on a daily basis. Also, applicants who did not reply to many of the question items described hereinafter ("no response" accounted for 12% or more of all the question items) were not subjected to analysis. Finally, the number of subjects to be analyzed was 25 (10 male subjects and 15 female subjects) and the average age was 42.4 years old (range: 22-68). The average sleep time of the subjects was 6.0 hours (self-reported over the past 1 month).

### Evaluation objects

Jelly was prepared using a hot-water extract extracted from dried *Hemerocallis fulva* var. *sempervirens* leaves by the method described in Example 1 and then used as a supplement for evaluation (hereinafter, described as a jelly extract). A small translucent portion made of PP was filled with a jelly extract and was then sealed with a transparent EP/DL/NY film. The jelly had a weight of about 17 g and contained 2 g of a hot-water extract extracted from dried *Hemerocallis fulva* var. *sempervirens* leaves. For comparison and contrast, a placebo jelly was also prepared without the addition of any hot-water extract, and then a portion of the same type described above was filled with jelly and then sealed. Aroma chemicals added to the 2 types of jelly were identical, but the jelly extract was high-fructose corn syrup and the placebo jelly had its taste adjusted with brown sugar. Differentiation between the two was difficult in terms of color and taste.

### Experimental techniques

Three (3) jelly extracts and 3 placebo jelly samples and instructions for implementing an experiment (denoted with "Test for Monitoring Sleep-Improving Supplement" for the subjects) and a set of questionnaires attached thereto were sent by mail to each subject's own home. Subjects were asked to ingest and evaluated jelly samples on any day.

This example was performed using a within-subjects design method. Specifically, all subjects were asked to experience both the jelly extract and the placebo jelly and then the evaluation results for each jelly sample were compared. For about a half of the subjects, the jelly extract was labeled with "supplement A" and the placebo jelly was labeled with "supplement B." For the remaining subjects, the jelly extract and the placebo jelly were labeled in the opposite manner (supplement B as supplement A, and vice versa). All subjects were instructed to ingest and evaluate supplement A and then supplement B at intervals of 2 or more days. The order of experiencing the 2 types of jelly was counter-balanced among subjects. The subjects were informed that the 2 types of jelly were both supplements developed for the purpose of sleep improvement. The same protocols were employed for the 2 instances of intake and evaluation of jelly samples. Regardless of the types of jelly, each subject was asked to: ingest 3 jelly samples as specified at about 2 hours before the planned bedtime; on the next day, respond to "the morning questionnaire" 30 minutes after waking up; and then respond to "the night questionnaire" 1 hour before bedtime in the night. The subjects were also asked to put all questionnaire forms into an envelop immediately after their responses, keep the forms therein, and never add to or alter their responses.

### Questionnaire items and questionnaire

### (1) To examine the sleep states of subjects for the Pittsburgh Sleep Quality Index (Japanese version) (Yuriko Doi, Masumi Minowa, Makoto Uchiyama, Kyoko Okawa (1998), Production of the Pittsburgh Sleep Quality Index (questionnaire), Japanese Version, Psychotherapeutics, 13(6), 761-763.) was used. Subjects were asked to respond to this questionnaire before one of the two instances of tasting jelly samples.

### (2) Morning questionnaire

Subjects were each asked to respond regarding sleep time during nights on which they took jelly samples, sleep quality, state of mind and/or emotion upon waking up, and sleep state. Regarding sleep time, subjects were each asked to enter sleep time to the minute in a predetermined column of a questionnaire form. Regarding sleep quality, subjects were each asked to respond using a VAS (Visual Analog Scale). Specifically, the left end of a 100-mm line segment drawn on the form was supposed to represent "very good" sleep, and the right end of the same was supposed to represent "very bad" sleep. Each subject was asked to express his or her own subjective evaluation of sleep quality during the previous night by providing a slit at any point on the line segment. After recovery of questionnaire forms, the length ranging from the left end of the line segment to the slit was measured to the millimeter as a score. Regarding measurement of state of mind and emotion, 8 items for the V (vitality) scale and 7 items for the F (fatigue) scale of the Japanese Version of POMS (Profile of Mood States) (McNaire, D. M., Lorr, M., & Droppleman, L. F., 1992 Profile of Mood States, San Diego: Educational and industrial testing service.; Kazuhito Yokoyama and Shunichi Araki (1997), Japanese Version, POMS Guidellines, KANEKOSHOBO) were used. Subjects were each asked to make a five-point evaluation (or asked to respond to each item, from "very applicable" (4 points) to "not applicable" (0 points). Also, regarding sleep state, subjects were each asked to make a five-point evaluation ranging from "very applicable" (4 points) to "not applicable" (0 points). Specifically, subjects were each asked to respond to 10 items for asking a subject's sleep quality or sleep state, including "could immediately fall asleep (could fall asleep immediately after going to bed)" and "experienced exhilaration upon waking up."

### (3) Night questionnaire

Subjects were each asked to respond to a questionnaire using VAS concerning physical conditions on the day after intake of a jelly sample followed by sleeping. The left end of a 100-mm line segment drawn on the form was supposed to represent "very good" and the right end of the same was supposed to represent "very bad." Each subject was asked to express and score his or her own subjective evaluation of physical conditions in the same manner as that used for sleep quality. Also, regarding daytime sleepiness, 8 situations were listed, including "while reading a book, a magazine, comics, a document, or the like" and "while taking a little rest after lunch." Subjects were each asked to make a four-point evaluation ranging from "never slept (probably)" (0 points), "did not sleep but sleepy (probably)" (1 point), "dozed off (probably)" (2 points), or "had a deep sleep (probably)" (3 points). Specifically, subjects were each asked to respond as to whether or not they actually felt sleepy in each situation, or, whether or not they experienced sleepiness if they were supposed to experience it.

### Results

The subjects in this example were recruited under conditions such that candidates were supposed to have subjective mild symptoms indicating sleep problems or sleep disorders, such as "having trouble getting to sleep," "awakening in the middle of night or early in the morning," or "having shallow sleep and still feeling fatigue during the next day," or candidates were supposed to be interested in sleep-improving supplements. Upon implementation of this experiment, subjects were asked to follow the rules regarding restrictions on smoking, taking of sleeping pills, and ingesting alcohol, food, and drinks containing caffeine.

### Sleep time, sleep quality, and physical conditions the next day

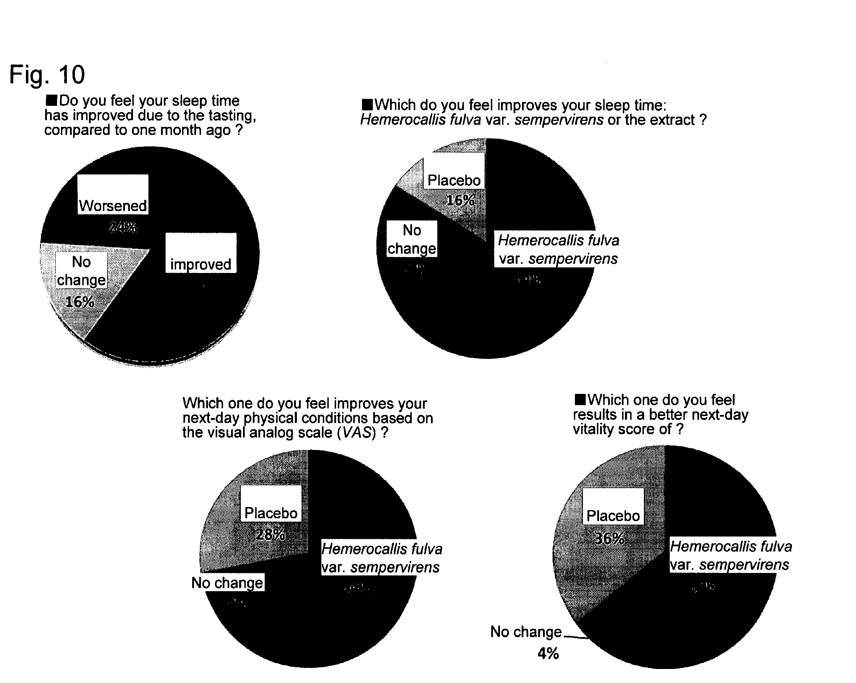

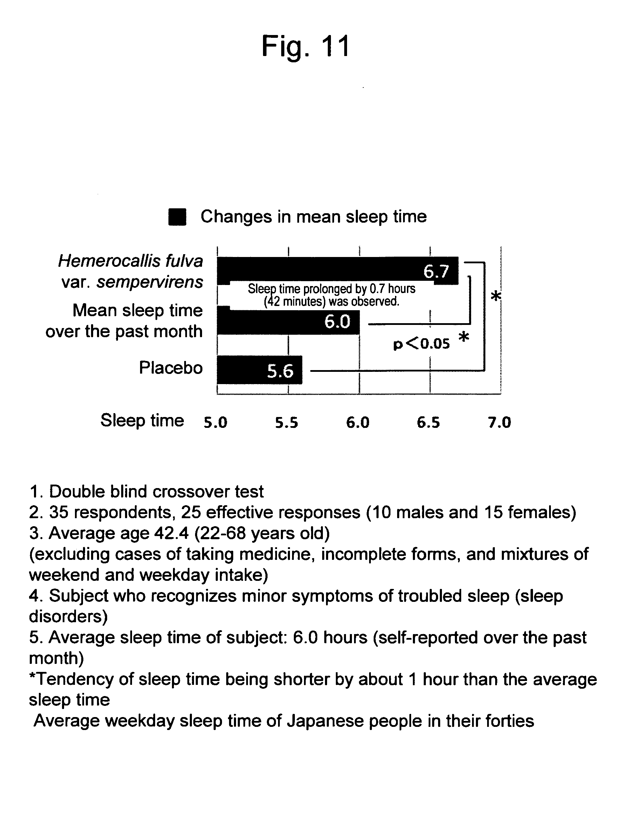

Table 1, Fig. 10, and Fig. 11 show sleep time, POMS scores, and scores for sleep quality and physical conditions measured by VAS for each type of condition. For reference, the average sleep time per day over the past month (obtained using responses to the Pittsburgh Sleep Quality Index (questionnaire)) is also shown in herein. While the average sleep time under conditions in which the placebo jelly had been taken was 5.6 hours (standard deviation = 1.75), the average sleep time under conditions in which the jelly extract had been taken was as long as 6.7 hours (standard deviation = 1.49). When the corresponding t-test was conducted, a significant difference (t(24) = 2.73, p<.05) was observed between conditions. Also, differences between the sleep time under conditions in which the jelly extract had been taken and the average sleep time over the past month were compared by a similar test and a significant difference was confirmed (t(24) = 2.23, p<.05). On the other hand, no statistically significant difference was detected (t(24)=1.16,n. s.) between the sleep time under conditions in which the placebo jelly had been taken and the average sleep time over the past month. Accordingly, sleep time was prolonged by the intake of the *Hemerocallis fulva* var. *sempervirens*-derived hot-water extract.

Meanwhile, regarding scores for sleep quality (t < 1) and physical conditions (t(24) = 1.26, n. s.), the value obtained under conditions in which the jelly extract had been taken was somewhat higher than the value under placebo conditions, but no significant difference was detected. Also, no difference was found in V scale (t(24) = 1.17, n. s.) in the POMS test. However, the F scale (score) (t(24)=1.77, p<.1) tended to show a significant difference between conditions, such that fatigue upon waking up was lowered by sleep after the intake of the hot-water extract.

### Table 1

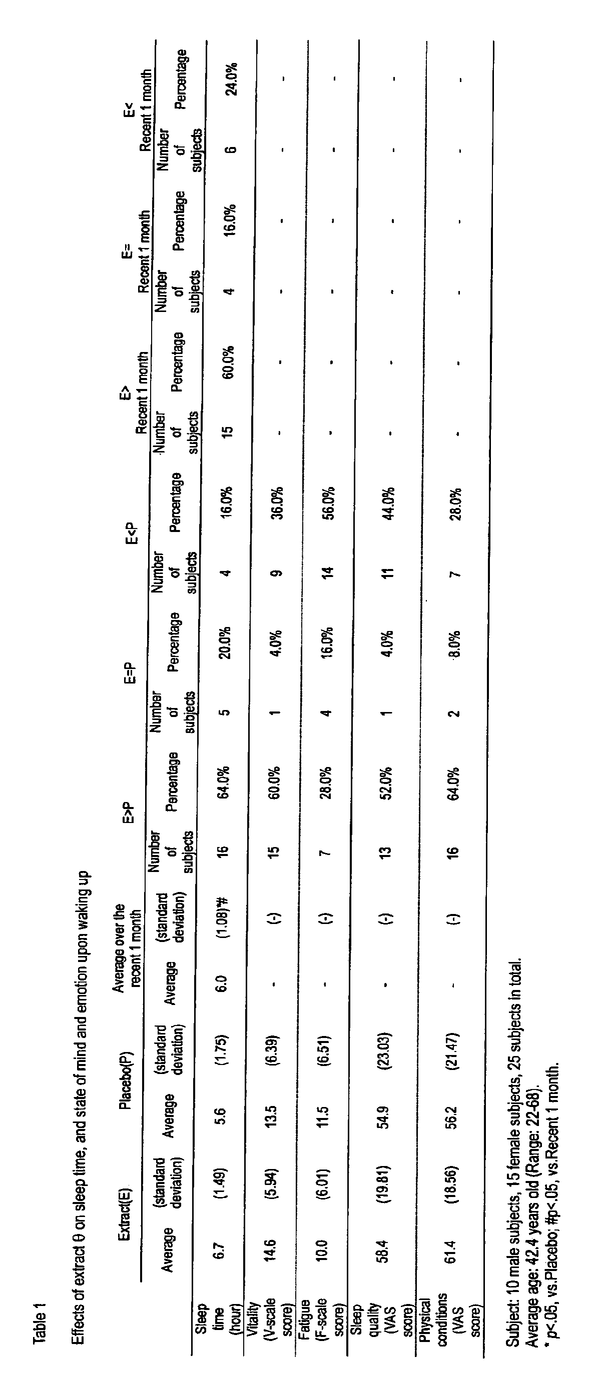

### Sleep state and the state of mind and emotion upon waking up

Subjects' responses to 10 items concerning the sleep state and the state of mind and emotion upon waking up (each subject was asked to respond to the "morning questionnaire") are summarized in Table 2 for each type of conditions. The corresponding t-test was conducted between conditions for all items. But no significant difference was detected for all items.

### Table 2

**Table 2**

| Effects of extract θ on sleep state, and state of mind and emotion upon waking up | | | | | | | | | | |
|---|---|---|---|---|---|---|---|---|---|---|
| | Extract(E) | | Placebo(P) | | E>P | | E=P | | E<P | |
| | Average | (standard deviation) | Average | (standard deviation) | Number of subjects | Percentage | Number of subjects | Percentage | Number of subjects | Percentage |
| Could fall asleep immediately after going to bed. | 2.40 | (1.19) | 2.20 | (1.26) | 10 | 40.0% | 8 | 32.0% | 7 | 28.0% |
| Awake in the middle of the night and then could not fall asleep. (*) | 1.12 | (0.93) | 1.24 | (1.23) | 6 | 24.0% | 13 | 52.0% | 6 | 24.0% |
| Awake at dawn and then could not fall asleep again (*) | 1.12 | (1.01) | 1.42 | (1.18) | 5 | 20.8% | 10 | 41.7% | 9 | 37.5% |
| Had a dream | 1.80 | (1.29) | 1.84 | (1.43) | 4 | 16.0% | 15 | 60.0% | 6 | 24.0% |
| A feeling of having slept deeply upon waking up | 2.16 | (0.85) | 1.92 | (1.22) | 10 | 40.0% | 10 | 40.0% | 5 | 20.0% |
| Exhilaration upon waking up | 1.92 | (0.86) | 1.64 | (1.04) | 9 | 36.0% | 11 | 44.0% | 5 | 20.0% |
| Felt fatigue remaining from yesterday upon waking up (*) | 1.96 | (1.10) | 1.80 | (1.00) | 7 | 28.0% | 13 | 52.0% | 5 | 20.0% |
| Felt motivated for work upon waking up (enthusiastic about work) | 1.88 | (1.01) | 1.88 | (0.88) | 6 | 24.0% | 14 | 56.0% | 5 | 20.0% |
| Felt a lack of sleep upon waking up (*) | 1.56 | (0.92) | 1.88 | (1.09) | 5 | 20.0% | 9 | 36.0% | 11 | 44.0% |
| Felt weak and heavy upon waking up (*) | 1.92 | (1.04) | 1.80 | (1.12) | 8 | 32.0% | 12 | 48.0% | 5 | 20.0% |

| | | | | | | | | | | |
|---|---|---|---|---|---|---|---|---|---|---|
| (*) Items reversed. Subject: 10 male subjects, 15 female subjects, 25 subjects in total. Average age: 42.4 years old (Range:22-68). | | | | | | | | | | |

### Daytime sleepiness during the next day

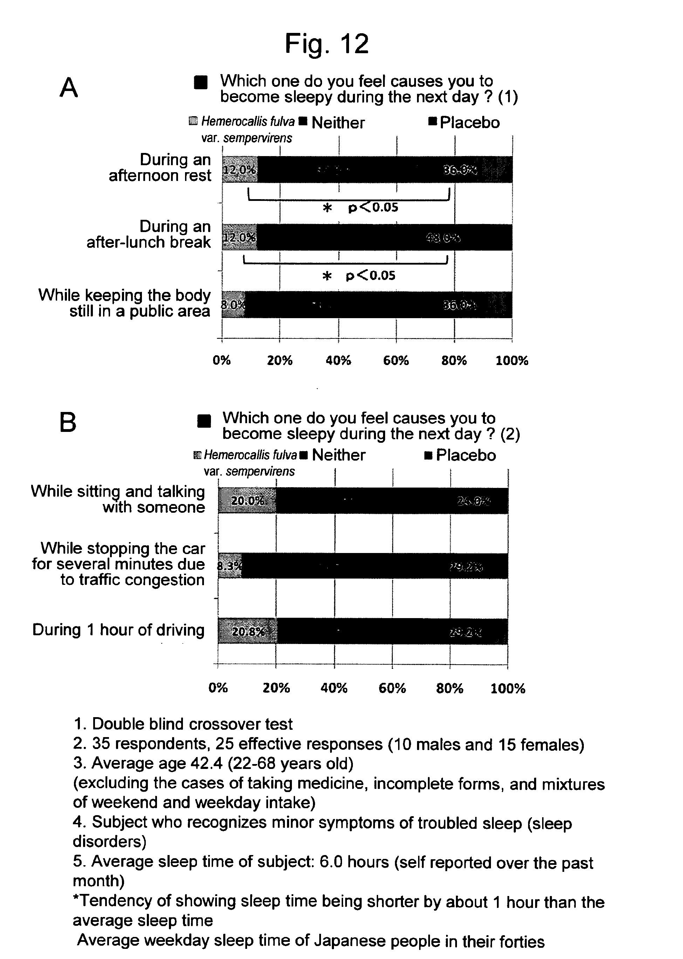

Subjects' responses to 8 items concerning daytime sleepiness (subjects were asked to respond to "night questionnaire") are summarized in Table 3, Figs. 12A and 12B for each type of conditions. In the 2 situations, "while keeping the body still in a public area" (t(24) = 2.28, p<.05) and "while taking a little rest after lunch" (t(24) = 2.79, p<.05), among the 8 situations, the value obtained under conditions in which the jelly extract had been taken was significantly lower than the value under the placebo conditions, indicating that daytime sleepiness during the next day was alleviated by the sleep after the intake of the hot-water extract.

### Table 3

**Table 3**

| Effects of extract θ on sleepiness in the next day | | | | | | | | | | |
|---|---|---|---|---|---|---|---|---|---|---|
| | Extract(E) | | Placebo(P) | | E>P | | E=P | | E<P | |
| | Average | (standard deviation) | Average | (standard deviation) | Number of subjects | Percentage | Number of subjects | Percentage | Number of subjects | Percentage |
| While reading a book, a magazine, comics, a document, or the like | 0.92 | (0.86) | 1.00 | (0.76) | 5 | 20.0% | 14 | 56.0% | 6 | 24.0% |
| While watching TV | 1.08 | (0.81) | 1.24 | (1.01) | 4 | 16.0% | 14 | 56.0% | 7 | 28.0% |
| While keeping the body still in a public area | 0.72 | (0.79) | 1.00 | (0.91)* | 2 | 8.0% | 14 | 56.0% | 9 | 36.0% |
| While sitting in a car for 1 hour. | 0.96 | (0.81) | 1.08 | (0.91) | 5 | 20.8% | 12 | 50.0% | 7 | 29.2% |
| While resting in the afternoon. | 1.52 | (1.12) | 1.80 | (0.76) | 3 | 12.0% | 13 | 52.0% | 9 | 36.0% |
| While sitting down and talking with someone | 0.32 | (0.56) | 0.32 | (0.56) | 5 | 20.0% | 14 | 56.0% | 6 | 24.0% |
| While taking a rest after lunch | 1.04 | (0.93) | 1.60 | (0.87)* | 3 | 12.0% | 10 | 40.0% | 12 | 48.0% |
| While stopping your car for several minutes due to traffic congestion | 0.33 | (0.64) | 0.56 | (0.71) | 2 | 8.3% | 15 | 62.5% | 7 | 29.2% |

| | | | | | | | | | | |
|---|---|---|---|---|---|---|---|---|---|---|
| Subject: 10 male subjects, 15 female subjects, 25 subjects in total Average age: 42.4 years old (Range: 22-68). * *P*<.05, vs. Placebo. | | | | | | | | | | |

### Example 8 Effects of a hot-water extract of Hemerocallis fulva var. sempervirens leaves on an electroencephalogram

Human subjects (Subject 1: 49-year-old female and Subject 2: 44-year-old male) were asked to ingest jelly samples containing a hot-water extract extracted from dried *Hemerocallis fulva* var. *sempervirens* leaves by the method described in Example 7. Subsequently, electroencephalograms were measured during sleep, so that changes over time in sleep stages were examined.

For controlled trial, subjects were asked to ingest portions of the same type filled with placebo jelly (not supplemented with a hot-water extract) sealed therein and then electroencephalograms were measured.

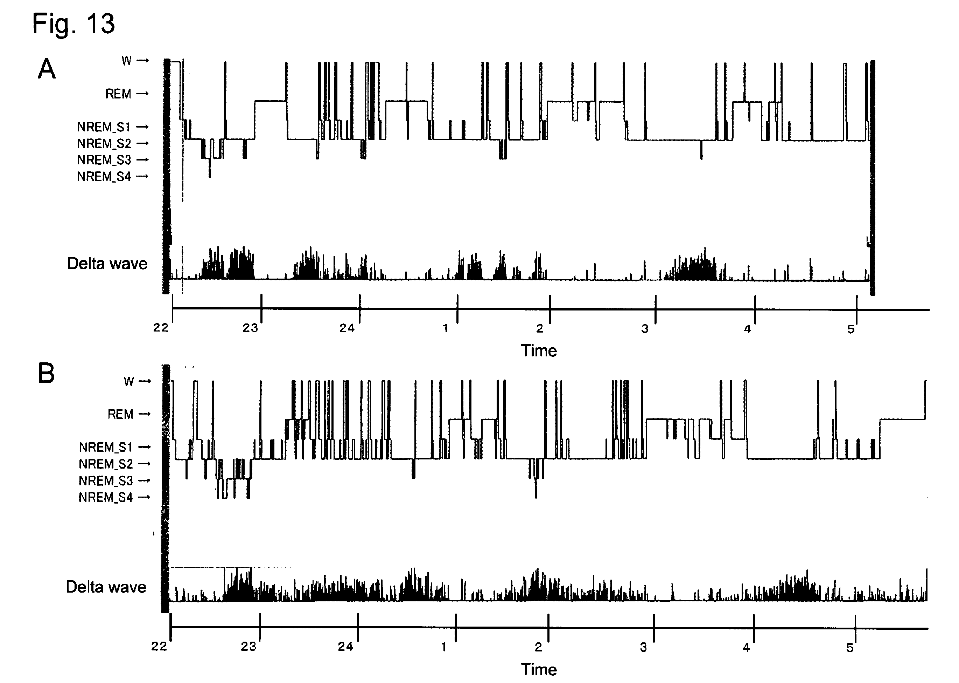

Fig. 13 shows the results for Subject 1. Fig. 13A shows an electroencephalogram measured when Subject 1 ingested a jelly sample (placebo) containing no extract. Fig. 13B shows an electroencephalogram measured when Subject 1 ingested a jelly sample containing the extract. The results of measuring electroencephalograms as shown in Fig. 13 revealed that: (i) the number of heavy deep sleep (NREM-S4) increased by 1 hour after bedtime; and (ii) the frequency (appearance) of delta wave (the frequency of electroencephalogram signals indicating deep sleep) increased throughout the time of sleep. This suggests that the intake of the extract resulted in an increase in deep sleep at the onset of sleep and increased the frequency of deep sleep during sleep.

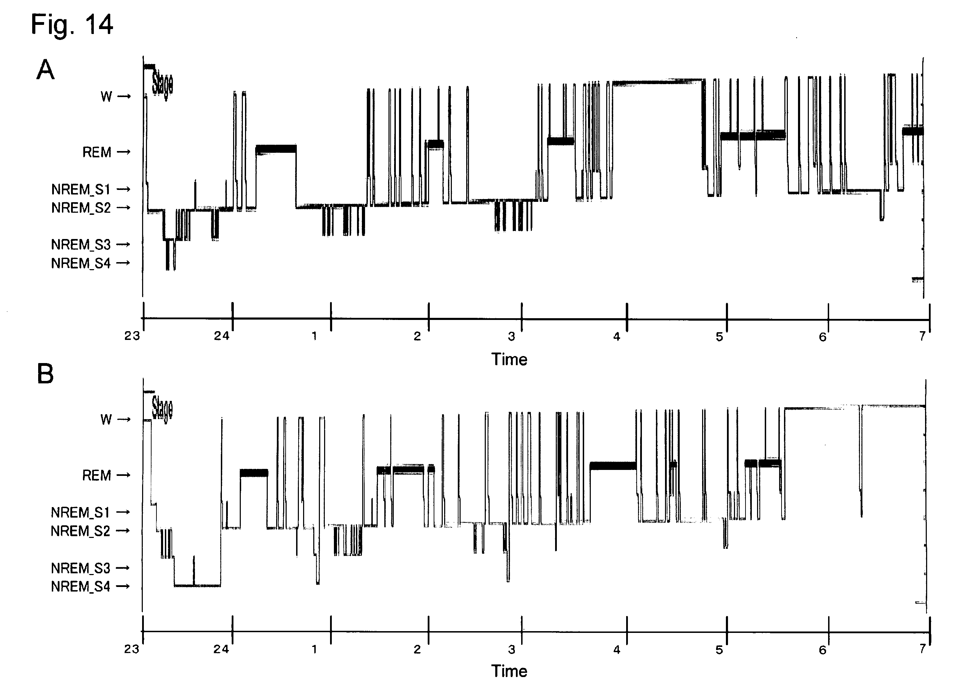

Fig. 14 shows the results for Subject 2. Fig. 14A shows an electroencephalogram measured when Subject 2 ingested a jelly sample (placebo) containing no extract. Fig. 14B shows an electroencephalogram measured when Subject 2 ingested a jelly sample containing the extract. The results of measuring electroencephalograms as shown in Fig. 14 revealed that: (i) the number of heavy deep sleep (NREM-S4) increased by 1 hour after bedtime and its duration is also prolonged; and (ii) the alert state (awakening) during 4 to 5 o'clock in the morning clearly disappeared as a result of the intake of the extract. This demonstrates that the intake of the extract increased deep sleep at the onset of sleep and further decreased awakening in the middle of the night.

### Example 9 Comparison of antidepressant-like effects and effects on deep body temperature change between the hot-water extracts of Hemerocallis fulva var. sempervirens and other substances

### 1. Antidepressant-like effects

An experimental apparatus (forced swimming apparatus) for determination of antidepressant-like effects used in Example 4 was used. Male C57BL/6N mice and male ddY mice that had experienced no experiment were bred in an animal room with a constant temperature and humidity for which a 12-12-hour light-dark cycle had been set. Mice were sufficiently acclimatized to breeding environment and then used as subject animals.

On day 1, forced swimming treatment was performed for 15 minutes. At 30 minutes after treatment, hot-water extracts extracted from dried *Hemerocallis fulva* var. *sempervirens* leaves and other substances were administered. Subsequently, on day 2, forced swimming treatment was performed for 5 minutes. At this time, at 30 minutes before treatment, the hot-water extracts extracted from dried *Hemerocallis fulva* var. *sempervirens* leaves or other substances were administered.

The time of immobility response during 5 minutes was measured using Super Mex Sensor (Muromachi Kikai Co., Ltd.) on day 1 and day 2.

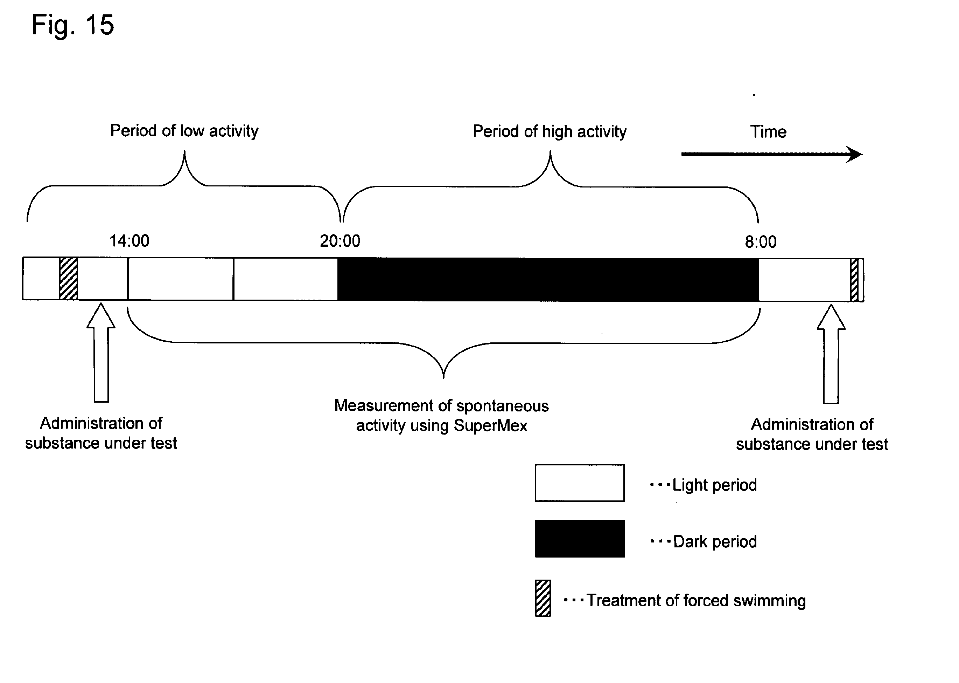

The experimental method is as shown in Fig. 15.

Substances used in this experiment are as follows.
(a) θ-high (a hot-water extract of *Hemerocallis fulva* var. *sempervirens* leaves)
(b) θ-low (a hot-water extract of *Hemerocallis fulva* var. *sempervirens* leaves)
(c) FX-high (a hot-water extract of *Hemerocallis fulva* var. *sempervirens* flowers)
(d) FX-low (a hot-water extract of *Hemerocallis fulva* var. *sempervirens* flowers)
(e) SJW (St. John's wort (*Hypericum perforatum*))
(f) Valleri (valerian)

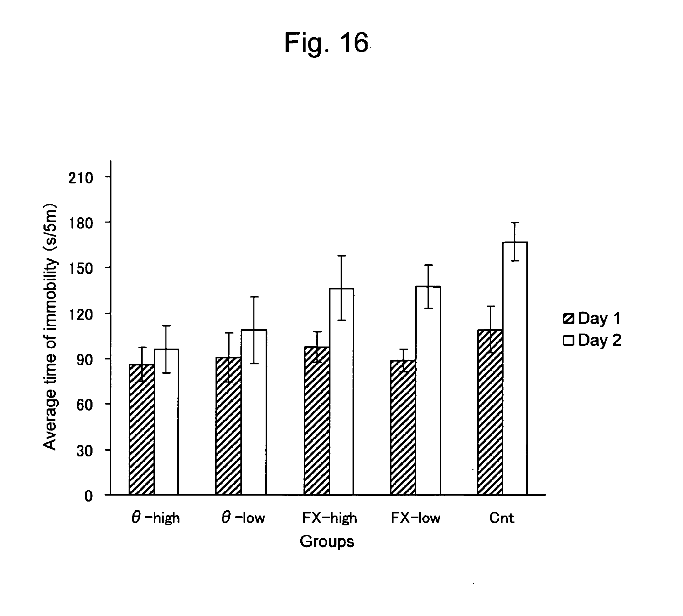

Fig. 16 shows the results of administering the above test articles (a), (b), (c), and (d). The results are respectively shown with the immobility response in each case.

As shown in Fig. 16, when the hot-water extracts of *Hemerocallis fulva* var. *sempervirens* leaves were used, prolongation of the time of immobility response was suppressed and when the hot-water extracts of *Hemerocallis fulva* var. *sempervirens* flowers were used, the time of immobility response was prolonged. The results demonstrate that antidepressant-like effects of the hot-water extracts of *Hemerocallis fulva* var. *sempervirens* leaves are superior to the same of *Hemerocallis fulva* var. *sempervirens* flowers.

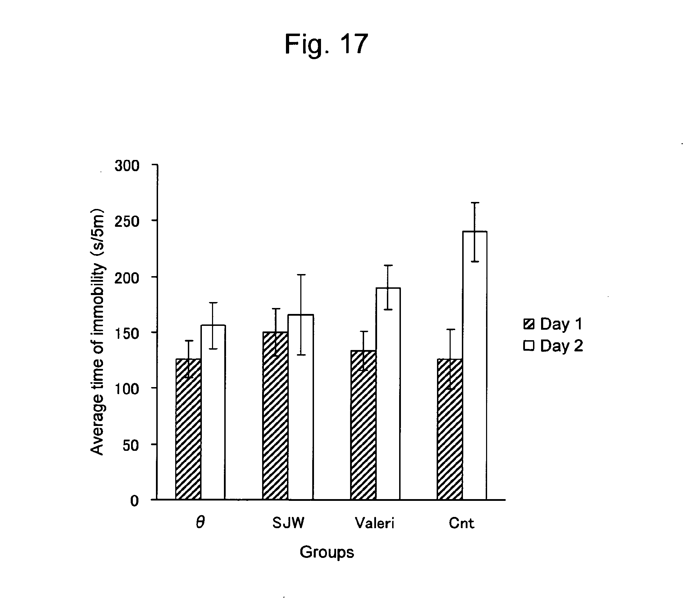

Fig. 17 shows the results of administering the above test articles (a), (e), and (f). The results are respectively shown with the immobility response in each case.

As shown in Fig. 17, the extract of *Hemerocallis fulva* var. *sempervirens* leaves exerted antidepressant-like effects equivalent to those of St. John's wort. On the other hand, valerian did not exert antidepressant-like effects.

### 2. Effects on deep body temperature

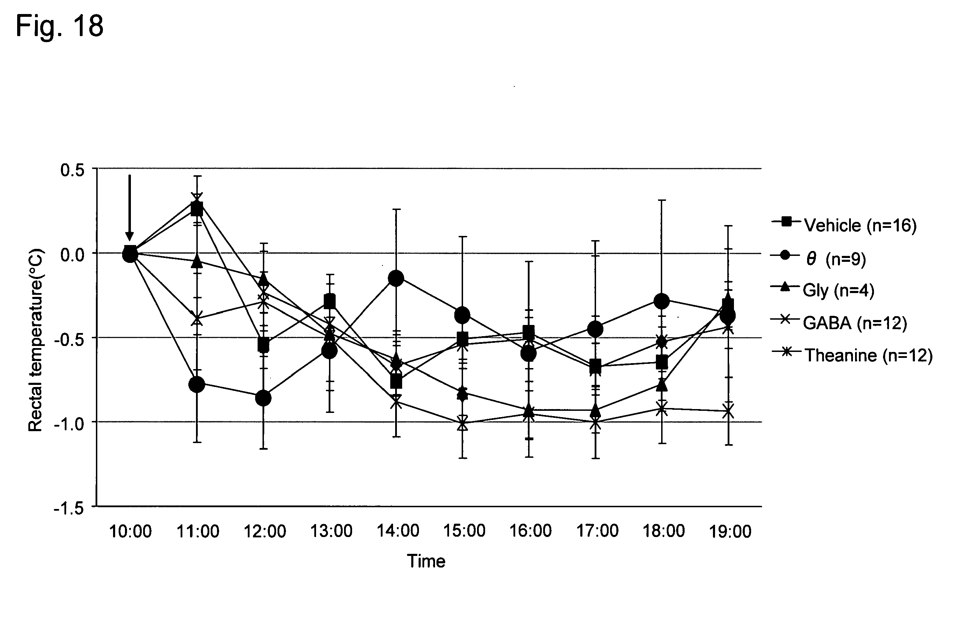

Fig. 18 shows the results of deep body temperature measurement using a hot-water extract of *Hemerocallis fulva* var. *sempervirens* leaves and other test substances. Skin capillary dilation in extremities was immediately confirmed in mice to which the hot-water extract (θ) of *Hemerocallis fulva* var. *sempervirens* leaves had been administered, and rectal temperature (deep body temperature) also decreased. The other test substances (Gly: glycine , GABA: γ-aminobutyric acid, and teanin) with the same concentration as that of the extract lowered the deep body temperature, but the rate of decrease was lower than that due to the hot-water extract of *Hemerocallis fulva* var. *sempervirens* leaves.

The results demonstrate that the hot-water extracts of *Hemerocallis fulva* var. *sempervirens* leaves have the highest effects of accelerating sleep among substances tested herein.

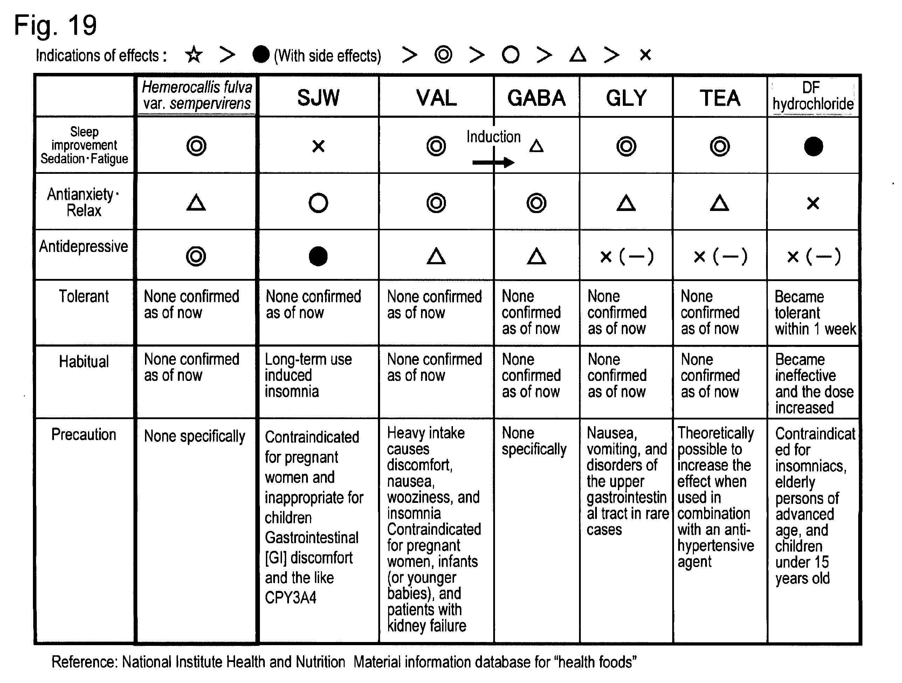

Fig. 19 shows the characteristics of *Hemerocallis fulva* var. *sempervirens* hot-water extracts and other sleep improvement-antianxiety-antidepressive-related substances. Characteristic of other sleep improvement·antianxiety·antidepressive-related substances are shown on the health food material database of the National Institute of Health and Nutrition.

### Industrial Applicability

It was confirmed as described in Examples that the hot-water extracts of a plant of the genus *Hemerocallis* exert antidepressant-like effects and fatigue-relieving effects through enhancement of sleep quality. It has never been discovered to date that extracts extracted using hot water from a plant of the genus *Hemerocallis* and particularly the extracts of the leaves thereof have antidepressant-like effects and fatigue-relieving effects based on sleep improvement. According to the present invention, antidepressant-like effects and fatigue-relieving effects based on sleep improvement of hot-water extracts of a plant of the genus *Hemerocallis* were demonstrated for the first time.

The hot-water extracts of a plant of the genus *Hemerocallis* of the present invention can be used in the fields of foods and beverages or medical fields as compositions having antidepressant-like effects and fatigue-relieving effects based on sleep improvement.

All publications, patents, and patent applications cited herein are incorporated herein by reference in their entirety.

## Claims

1. A composition having antidepressant-like effects, which contains a hot-water extract of a plant of the genus *Hemerocallis* as an active ingredient.

2. The composition having antidepressant-like effects according to claim 1, wherein the plant of the genus *Hemerocallis* is *Hemerocallis fulva* var. *sempervirens.*

3. The composition having antidepressant-like effects according to claim 1 or 2, wherein the hot-water extract is a hot-water extract of leaves of a plant of the genus *Hemerocallis.*

4. The composition having antidepressant-like effects according to any one of claims 1 to 3, which is a food or a beverage.

5. The composition having antidepressant-like effects according to claim 4, which is labeled with its intended use such that it is used for alleviating depressive symptoms.

6. A composition having fatigue-relieving effects based on sleep improvement, which contains a hot-water extract of a plant of the genus *Hemerocallis* as an active ingredient.

7. The composition having fatigue-relieving effects based on sleep improvement according to claim 6, wherein a plant of the genus *Hemerocallis* is *Hemerocallis fulva* var. *sempervirens.*

8. The composition having fatigue-relieving effects based on sleep improvement according to claim 6 or 7, wherein the hot-water extract is a hot-water extract of leaves of a plant of the genus *Hemerocallis.*

9. The composition having fatigue-relieving effects based on sleep improvement according to any one of claims 6 to 8, which is a food or a beverage.

10. The composition having fatigue-relieving effects based on sleep improvement according to claim 9, which is labeled with its intended use such that it is used for relieving fatigue.

11. A method for producing a composition having antidepressant-like effects and fatigue-relieving effects based on sleep improvement from a plant of the genus *Hemerocallis,* which comprises
heating a dried plant of the genus *Hemerocallis* with water at 60°C to 100°C for 30 to 90 minutes, filtering the thus obtained extract, and then concentrating the filtrate at 0.5 ml to 2 ml per gram of the dried plant of the genus *Hemerocallis.*

12. The method according to claim 11, which further comprises adding 1 to 3 volumes of a water soluble organic solvent to 1 volume of a filtered extract so that the concentration of the organic solvent ranges from 55% to 90%, filtering the resultant, and then concentrating the filtrate to 0.5 ml to 2 ml per gram of the dried plant of the genus *Hemerocallis.*
